# EUROPÄISCHE PATENTSCHRIFT

(11) **EP 3 621 941 B1**
(45) Veröffentlichungstag und Bekanntmachung des Hinweises auf die Patenterteilung: **06.10.2021**
(21) Anmeldenummer: 18724833.1
(22) Anmeldetag: 09.05.2018
(51) Int. Cl.: C07B 59/00, C07C 253/30, C07C 313/06, C07C 227/02

(54) **VERWENDUNG VON PRÄKURSOREN FÜR DIE HERSTELLUNG VON KOHLENSTOFF-11-MARKIERTEN AMINOSÄUREN UND DERIVATEN DAVON**
USE OF PRECURSORS FOR THE PRODUCTION OF CARBON-11-LABELLED AMINO ACIDS AND DERIVATIVES THEREOF
UTILISATION DE PRÉCURSEURS POUR LA PRÉPARATION D'ACIDES AMINÉS MARQUÉS AU CARBONE 11 ET LEURS DÉRIVÉS

(30) Priorität: 12.05.2017 DE 102017110429
(43) Veröffentlichungstag der Anmeldung: 18.03.2020
(73) Patentinhaber: ABX Advanced Biochemical Compounds GmbH, 01454 Radeberg (DE)
(72) Erfinder: HESSE, Ronny, 01069 Dresden (DE); FASEL, Antje, 01097 Dresden (DE); BUGDAHN, Nikolas, 37603 Holzminden (DE); MÜLLER, Marco, 01324 Dresden (DE); HOEPPING, Alexander, 01324 Dresden (DE)
(74) Vertreter: Riechelmann & Carlsohn Patentanwälte PartG mbB
(86) Internationale Anmeldenummer: PCT/EP2018/062147
(87) Internationale Veröffentlichungsnummer: WO 2018/206728

(56) Entgegenhaltungen:
- JUNHAO XING ET AL: "High-Yielding Automated Convergent Synthesis of No-Carrier-Added [11C-Carbonyl]-Labeled Amino Acids Using the Strecker Reaction", SYNLETT, Bd. 28, Nr. 03, 7. November 2016 (2016-11-07), Seiten 371-375, XP055483195, DE ISSN: 0936-5214, DOI: 10.1055/s-0036-1588638 in der Anmeldung erwähnt
- PRASAD B A B ET AL: "Trimethylsilyl cyanide addition to aldimines and its application in the synthesis of (S)-phenylglycine methyl ester", TETRAHEDRON LETTERS, ELSEVIER, AMSTERDAM, NL, Bd. 45, Nr. 52, 20. Dezember 2004 (2004-12-20), Seiten 9565-9567, XP027297918, ISSN: 0040-4039 [gefunden am 2004-11-26]

## Beschreibung

Die Erfindung betrifft die Verwendung von Präkursoren für die Herstellung von Kohlenstoff-11-markierten Aminosäuren und Aminosäure-Derivaten sowie ein Verfahren zur Herstellung von Kohlenstoff-11-markierten Aminosäuren und Aminosäure-Derivaten unter Verwendung dieser Präkursoren.

Mit Kohlenstoff-11-radiomarkierte Verbindungen werden als Radiopharmazeutika in der Positronenemissionstomografie (PET) verwendet. Sie können dabei in der onkologischen Bildgebung als Tracer eingesetzt werden. Sie werden auch in der Neuro-Onkologie verwendet. Eine wichtige Gruppe dieser radiomarkierten Verbindungen sind Kohlenstoff-11-radiomarkierte Aminosäuren. Diese Aminosäuren gehören zu den chiralen PET-Radiopharmazeutika. Sie können beispielsweise verwendet werden, um den erhöhten Aminosäuremetabolismus, der für Krebszellen beschrieben wird, darzustellen. Die radioaktive Markierung von Aminosäuren mit Kohlenstoff-11 bietet den Vorteil, dass die radiomarkierte Aminosäure und das endogene Molekül eine identische Struktur aufweisen. Im Vergleich zu Fluorid-18-markierten Aminosäuren ist der Aufwand, um Kohlenstoff-11 markierte Aminosäure in der klinischen Praxis einzusetzen zu können, deutlich geringer, weil die dazu jeweils notwendigen pharmakologischen und toxikologischen Tests weniger Aufwand erfordern. Dieser Umstand zeigt sich beispielsweise im Vergleich von [¹¹C]Cholin, das eine Kohlenstoff-11-markierte Variante der endogenen Verbindung Cholin ist, und seinem fluorierten Analogon, [¹⁶F]Fluorcholin.

Mit variablen Erfolgen und oft in asymmetrischer Weise wurden viele Kohlenstoff-11-Aminosäure-Isotopologen hergestellt, bei denen Kohlenstoff-12 mit radioaktivem Kohlenstoff-11 substituiert ist. Die größte Herausforderung bei der Synthese von Kohlenstoff-11-markierten Aminosäuren liegt immer noch in der stereoselektiven Reaktion am α-Kohlenstoff zur Vermeidung zeitraubender chiraler Trennung via HPLC, um die enantiomerenreine Form einfach und zuverlässig aus dem Radiomarkierungsprozess zu erhalten, welcher selbst schon durch die Synthesezeit und die spezifische Aktivität begrenzt ist.

Die Bedeutung der Enantiomerenreinheit von Radiopharmaka ist für radioaktiv markierte Aminosäuren von größter Bedeutung, da die Stereochemie die Geschwindigkeit und Selektivität des Aminosäuretransports beeinflusst. Aus diesem Grund werden L-Enantiomere in Säugetierzellen bevorzugt, wie durch die Anwendung von L- und D-[¹¹C]Phenylalanin gezeigt werden konnte, wobei das L-Enantiomer von [¹¹C]Phenylalanin bessere Pankreas-zu-Leber-Verhältnisse zeigte. Dies wurde auch noch durch die besseren bilderzeugenden Eigenschaften von L-¹⁸F-markierten Fluoralkylphenylalanin-Analoga nachgewiesen, die alle eine hohe Tumoraufnahme im Vergleich zum umliegenden Gewebe zeigen. Diese Studien, ohne näher auf die Goldstandards L-[¹¹C]Methionin und L-[¹⁸F]Fluorethyltyrosin einzugehen, betonen daher die Notwendigkeit, Methoden zur Synthese von enantiomerenreinen Aminosäuren als PET-Tracer zu entwickeln.

Darüber hinaus ermöglicht die Halbwertszeit von Kohlenstoff-11 (20,39 min), dass bei einem Patienten am selben Tag bei einem einzigen Krankenhausbesuch Untersuchungen mit verschiedenen [¹¹C]markierten PET-Radiotracern durchgeführt werden können. Die Halbwertszeit bedeutet allerdings auch, dass der Zeitraum, der für die Herstellung und Anwendung des [¹¹C]markierten PET-Radiotracers zur Verfügung steht, äußert kurz ist. Zeitaufwendige Synthesen verringern die praktische Anwendbarkeit eines [¹¹C]markierten PET-Radiotracers daher erheblich. In der Regel stehen für die Synthese, Reinigung und Qualitätskontrolle eines [¹¹C]markierten PET-Radiotracers nur 60 min zur Verfügung. Das heißt, die Anzahl der Syntheseschritte muss so gering wie möglich sein. Außerdem muss die für einen Syntheseschritt erforderliche Reaktionszeit so kurz wie möglich sein.

Zur Synthese Kohlenstoff-11-markierter Aminosäuren kann die Strecker-Reaktion eingesetzt werden (siehe Xing, J. et al., High-yielding automated convergent synthesis of no-carrier-added [11C-carbonyl]-labeled amino acids using the strecker reaction. Synlett (2017), 28(3), 371-375). Die von Xing et al. vorgeschlagene Variante der Strecker-Reaktion für die Herstellung von [¹¹C]Sacrosin, wobei das Kohlenstoffatom der Carbonylgruppe das Kohlenstoff-11-Atom ist, erfordert die Herstellung von [¹¹C]α-Aminonitril durch Kondensation von Formaldehyd mit Methylamin und [¹¹C]Natriumcyanid. Das so erhaltene Aminonitril wird anschließend einer basischen Hydrolyse mit Natriumhydroxid unterzogen. Diese Variante kann auf andere [¹¹C]Aminosäuren übertragen werden, wobei das Formaldehyd durch ein anderes Keton oder das Methylamin durch ein anderes Amin oder beide Verbindungen ersetzt werden müssen. Die erforderlichen Synthesezeiten liegen unter 20 min. Die radioaktiven Ausbeuten sind jedoch gering. Darüber hinaus gibt es in diesem Verfahren keine Möglichkeit, Aminosäuren enantioselektiv herzustellen. Prasad, B., et al. (Tetrahedron Letters (2004) 45, 9565-9567) beschreiben die Addition von Trimethylsilylcyanid an Arylaldimine als Form der Strecker-Synthese.

Aufgabe der Erfindung ist es, die Nachteile nach dem Stand der Technik zu beseitigen. Es sollen insbesondere eine Verwendung von Präkursoren zur enantioselektiven Herstellung von Aminosäuren und Derivaten davon vorgeschlagen werden. Ferner soll ein Verfahren zur diastereoselektiven Markierung dieser Präkursoren mit Kohlenstoff-11 vorgeschlagen werden.

Diese Aufgabe wird durch die Merkmale der Ansprüche 1 und 5 gelöst. Zweckmäßige Ausgestaltungen der Erfindungen ergeben sich aus den Merkmalen der Unteransprüche.

Nach Maßgabe der Erfindung ist die Verwendung eines Präkursors für die Herstellung von Kohlenstoff-11 markierten Aminosäuren oder Derivaten vorgesehen. Der Präkursor ist eine Verbindung der Formel I: 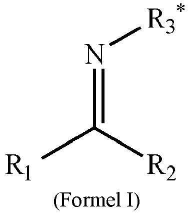 wobei
R₁ und R₂ unabhängig voneinander aus der Gruppe ausgewählt sind, die Wasserstoff, unsubstituiertes oder substituiertes C₁-C₆-Alkyl, das gegebenenfalls durch Einlagerung zumindest einer Gruppe X in die Kohlenstoffkette modifiziert sein kann, unsubstituiertes oder substituiertes C₂-C₆-Alkenyl, das gegebenenfalls durch Einlagerung zumindest einer Gruppe X in die Kohlenstoffkette modifiziert sein kann, substituiertes oder unsubstituiertes Alkylaryl, substituiertes oder unsubstituiertes Arylalkyl substituiertes oder unsubstituiertes Aryl und substituiertes oder unsubstituiertes Heteroaryl umfasst; wobei R₁ vorzugsweise unsubstituiertes oder substituiertes C₁-C₆-Alkyl, substituiertes oder unsubstituiertes Aryl oder substituiertes oder unsubstituiertes Heteroaryl, stärker bevorzugt substituiertes oder unsubstituiertes C₁-C₆-Alkyl oder substituiertes oder unsubstituiertes Aryl und besonders bevorzugt unsubstituiertes oder substituiertes C₁-C₆-Alkyl ist; und R₂ vorzugsweise unsubstituiertes oder substituiertes C₁-C₆-Alkyl oder Wasserstoff, besonders bevorzugt Wasserstoff ist;
R₃ ein chirales Auxilar ist, das aus der Gruppe ausgewählt ist, die substituiertes oder unsubstituiertes C₁-C₆-Alkylsulfinyl, substituiertes oder unsubstituiertes Arylsulfinyl, substituiertes oder unsubstituiertes Arylalkyl und substituiertes oder unsubstituiertes Arylglycinol umfasst; wobei R₃ vorzugsweise substituiertes oder unsubstituiertes C₁-C₆-Alkylsulfinyl ist;
X aus der Gruppe ausgewählt ist, die Sauerstoff, Schwefel, -SO-, -SO₂- und -N(R₁₀)- umfasst;
R₁₀ Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst und
die Reste gegebenenfalls ungeschützt oder geschützt sind. Insbesondere können R₁ und/oder R₂ ungeschützt oder geschützt und die anderen Reste ungeschützt sein.

Der erfindungsgemäße Präkursor ermöglicht die diastereoselektive Markierung mit einem Kohlenstoff-11-Synthon, d. h. es wird ein Diastereomer bevorzugt erhalten. Durch die anschließende Abspaltung des chiralen Auxiliars erlaubt der Präkursor somit die enantioselektive Herstellung von Kohlenstoff-11-markierten Aminosäuren und Derivaten davon. Der erfindungsgemäße Präkursor kann insbesondere zur enantioselektiven Herstellung von α-Aminosäuren und Derivaten davon wie α-Aminosäure-Ester und α-Aminosäure-Amide, besonders bevorzugt L-α-Aminosäuren und Derivaten davon wie L-α-Aminosäure-Ester und L-α-Aminosäure-Amide verwendet werden. Der erfindungsgemäße Präkursor kann aber auch zur enantioselektiven Herstellung von D-α-Aminosäuren und Derivaten davon wie D-α-Aminosäure-Ester und D-α-Aminosäure-Amide verwendet werden. Der erfindungsgemäße Präkursor ermöglicht somit die enantioselektive Herstellung entweder der L-α-Aminosäuren und Derivaten davon wie L-α-Aminosäure-Ester und L-α-Aminosäure-Amide oder der D-α-Aminosäuren und Derivaten davon wie D-α-Aminosäure-Ester und D-α-Aminosäure-Amide.

Die Präkursoren der Formel I sollten thermodynamisch stabile Verbindungen sein.

Der Ausdruck "substituiert" bezieht sich in der vorliegenden Erfindung insbesondere auf einen oder mehrere Substituenten, die aus der Gruppe ausgewählt sind, die Halogen, Cyano, Nitro, geschütztes oder ungeschütztes Hydroxy, geschütztes oder ungeschütztes -N(R₁₁R₁₂) und geschütztes oder ungeschütztes Thiol umfassen. R₁₁ und R₁₂ können unabhängig voneinander aus der Gruppe ausgewählt sein, die Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst. R₁₁ und R₁₂ sind vorzugsweise beide Wasserstoff.

Der Ausdruck "geschützt" bezieht sich insbesondere auf eine Schutzgruppe, zum Schutz der Hydroxy-Gruppe, der -N(R₁₁R₁₂)-Gruppe oder der Thiolgruppe. Die Schutzgruppe kann nach Umsetzung des Präkursors entfernt werden. Zum Schutz der Hydroxy-Gruppe können bekannte Schutzgruppen verwendet werden. Eine Schutzgruppe zum Schutz der Hydroxy-Gruppe kann beispielsweise aus der Gruppe ausgewählt sein, die Trityl, Benzyl, Methoxybenzyl, p-Nitrobenzyl, Benzoyl, substituiertes Benzoyl, Trimethylsilyl, Triethylsilyl, Isopropyldimethylsilyl, tert-Butyldimethylsilyl, tert-Butyldiphenylsilyl, Thexyldimethylsilyl, Allyl, Methoxymethyl, (2-Methoxyethoxy)methyl und Tetrahydropyranyl umfasst. Zum Schutz der Amin-Gruppe, d. h. R₁₁ und R₁₂ sind beide Wasserstoff, können bekannte Schutzgruppen verwendet werden. Eine Schutzgruppe zum Schutz der Amin-Gruppe kann beispielsweise aus der Gruppe ausgewählt sein, tert-Butylcarbonyl, Benzylcarbonyl, 9-Fluorenylmethylcarbonyl und Allylcarbonyl umfasst. Zum Schutz der Thiol-Gruppekönnen bekannte Schutzgruppen verwendet werden.

Der Ausdruck "Halogene" bezieht sich auf Fluor, Chlor, Brom oder Iod.

Der Ausdruck "Alkyl" bezieht sich insbesondere auf eine gesättigte aliphatische Kohlenwasserstoff-Gruppe mit einer verzweigten oder unverzweigten Kohlenstoffkette mit 1 bis 6 Kohlenstoffatomen.

Der Ausdruck "Alkylen" bezieht sich insbesondere auf eine ungesättigte aliphatische Kohlenwasserstoff-Gruppe mit 2 bis 6 Kohlenstoffatomen, beispielsweise Ethylen, 2,2-Dimethylethylen, Propylen, 2-Methylpropylen, Butylen, Pentylen und dergleichen.

Der Ausdruck "Aryl" bezieht sich auf eine einwertige cyclische aromatische Kohlenwasserstoff-Gruppe, die einen mono-, bi- oder tricyclischen aromatischen Ring umfassen kann. Die Arylgruppe kann gegebenenfalls substituiert sein. Beispiele von Arylgruppen sind - gegebenenfalls substituiertes - Phenyl, Naphthyl, Phenanthryl, Fluorenyl, Indenyl, Azulenyl, Oxydiphenyl, Biphenyl, Methylendiphenyl, Aminodiphenyl, Diphenylsulfidyl, Diphenylsulfonyl, Diphenylisopropylidenyl, Benzodioxanyl, Benzodioxylyl, Benzoxazinyl, Benzoxazinonyl, Benzopiperadinyl, Benzopiperazinyl, Benzopyrrolidinyl, Benzomorpholinyl, Methylendioxyphenyl, Ethylendioxyphenyl und dergleichen, wobei die Aufzählung nicht vollständig ist. Vorzugsweise umfasst Aryl gegebenenfalls substituiertes Phenyl und gegebenenfalls substituiertes Naphthyl.

Der Ausdruck "Heteroaryl" bezieht sich insbesondere auf eine monocyclische, bicyclische oder tricyclische Gruppe mit 5 bis 12 Ringatomen, wobei mindestens ein aromatischer Ring ein, zwei oder drei Ringheteroatome, ausgewählt aus N, O oder S, enthält, wobei die verbleibenden Ringatome C sind. Die Heteroaryl-Gruppe kann gegebenenfalls substituiert sein. Beispiele von Heteroaryl-Gruppen sind - gegebenenfalls substituiertes - Imidazolyl, Oxazolyl, Isoxazolyl, Thiazolyl, Isothiazolyl, Oxadiazolyl, Thiadiazolyl, Pyrazinyl, Pyridazinyl, Thiophenyl, Furanyl, Pyranyl, Pyridinyl, Pyrrolyl, Pyrazolyl, Pyrimidyl, Chinolinyl, Isochinolinyl, Chinazolinyl, Benzofuranyl, Benzothiophenyl, Benzothiopyranyl, Benzimidazolyl, Benzoxazolyl, Benzooxadiazolyl, Benzothiazolyl, Benzothiadiazolyl, Benzopyranyl, Indolyl, Isoindolyl, Indazolyl, Triazolyl, Triazinyl, Chinoxalinyl, Purinyl, Chinazolinyl, Chinolizinyl, Naphthyridinyl, Pteridinyl, Carbazolyl, Azepinyl, Diazepinyl, Acridinyl und dergleichen, wobei die Aufzählung nicht vollständig ist.

Der Ausdruck "Alkylaryl" bezieht sich insbesondere auf einwertige, eine Arylgruppe tragende Alkylreste, wobei der Alkylrest, wie oben definiert, 1 bis 6 Kohlenstoffatome aufweisen kann.

Der Ausdruck "Arylalkyl" bezieht sich insbesondere auf einwertige, eine Alkylgruppe tragende Arylreste, wobei der Alkylrest, wie oben definiert, 1 bis 6 Kohlenstoffatome aufweisen kann.

Vorzugsweise ist R₁ aus der Gruppe ausgewählt, die Methyl, geschütztes und ungeschütztes Hydroxymethyl, Ethyl, n-Propyl, iso-Propyl, n-Butyl, iso-Butanyl, sec-Butyl, tert-Butyl, Phenyl, geschütztes und ungeschütztes Hydroxyphenyl, Benzyl und geschütztes und ungeschütztes Hydroxybenzyl umfasst, und ist R₂ Wasserstoff.

Der Ausdruck "chirales Auxiliar" bezieht sich insbesondere auf eine Gruppe mit zumindest einem Chiralitätszentrum. Das chirale Auxiliar ermöglicht eine diastereoselektive Umsetzung des erfindungsgemäßen Präkursors, so dass nach der Abspaltung des chiralen Auxiliars ein Enantiomer bevorzugt erhalten wird. Bevorzugte chirale Auxiliare sind substituiertes oder unsubstituiertes Alkylsulfinyl und substituiertes oder unsubstituiertes Arylsulfinyl, wobei substituiertes oder unsubstituiertes Alkylsulfinyl besonders bevorzugt ist. Aufgrund des chiralen Auxiliars weist der Präkursor der Formeln I zwei Enantiomere auf. Die beiden Enantiomere sind in den Formel I-a und Ib veranschaulicht, wobei die Darstellung der Bindung zwischen dem Stickstoffatom und der Gruppe R₃ lediglich die beiden Enantiomere veranschaulichen soll, aber nicht zwingend den Projektionsregeln entspricht, weil diese vom Aufbau des Restes R₃ abhängt. Formel I-a veranschaulicht ein Enantiomer, das bevorzugt zur Herstellung der D-Aminosäure verwendet werden kann. Formel I-b veranschaulicht ein Enantiomer, das bevorzugt zur Herstellung der L-Aminosäure verwendet werden kann: 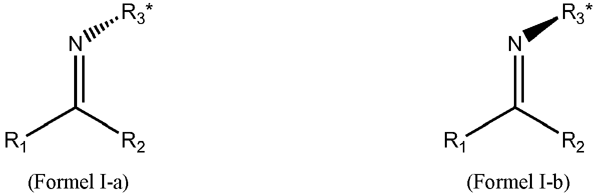 wobei R₁, R₂ und R₃ wie oben definiert sind. Je nach chiralem Auxiliar und Reaktionsbedingung wird entweder die L- oder die D-Aminosäure bevorzugt gebildet.

Die als Alkylsulfinyl und Arylsulfinyl bezeichneten chiralen Auxiliare stellen eine -S(O)-R₄-Gruppe dar, wobei R₄ aus der Gruppe ausgewählt ist, die unsubstituiertes oder substituiertes C₁-C₆-Alkyl und substituiertes oder unsubstituiertes Aryl umfasst. In diesem Fall ist die Verbindung der Formel I eine Verbindung der Formel II-a oder II-b: 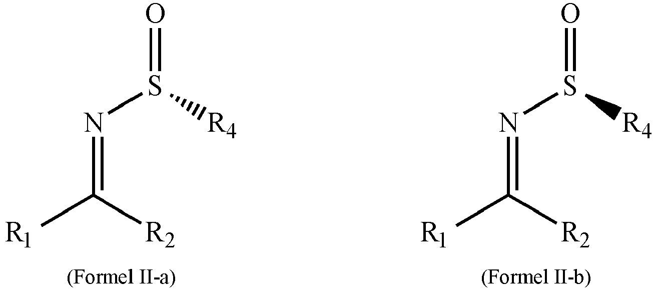 wobei R₁ und R₂ wie oben definiert sind und R₄ aus der Gruppe ausgewählt ist, die unsubstituiertes oder substituiertes C₁-C₆-Alkyl und substituiertes oder unsubstituiertes Aryl umfasst. Bevorzugt ist R₄ *tert*.-Butyl. Für Herstellung von L-Aminosäuren und ihren Derivaten wird vorzugsweise eine Verbindung der Formel II-b verwendet.

In der bevorzugten Ausführungsform ist der erfindungsgemäße Präkursor eine Verbindung der Formel II-a oder II-b, wobei R₁ aus der Gruppe ausgewählt ist, die unsubstituiertes oder substituiertes C₁-C₆-Alkyl, substituiertes oder unsubstituiertes Aryl und substituiertes oder unsubstituiertes Heteroaryl umfasst; R₂ Wasserstoff ist; und R₄ aus der Gruppe ausgewählt ist, die unsubstituiertes oder substituiertes C₁-C₆-Alkyl und substituiertes oder unsubstituiertes Aryl umfasst. In einer stärker bevorzugten Ausführungsform ist der erfindungsgemäße Präkursor eine Verbindung der Formel II-a oder II-b, wobei R₁ aus der Gruppe ausgewählt sind, die unsubstituiertes oder substituiertes C₁-C₆-Alkyl, substituiertes oder unsubstituiertes Aryl und substituiertes oder unsubstituiertes Heteroaryl umfasst; R₂ Wasserstoff ist; und R₄ *tert*.-Butyl ist. Ein derartiger Präkursor ist eine Verbindung der Formel III-a oder III-b:

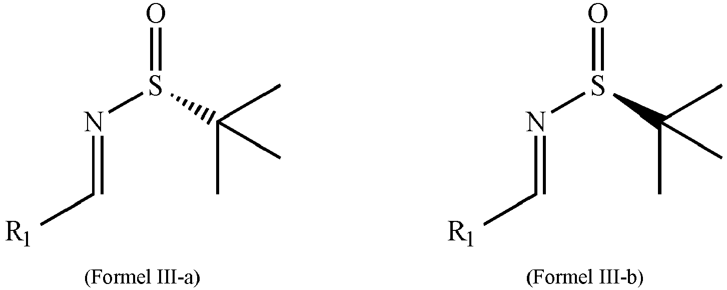

In einer stärker bevorzugten Ausführungsform ist der erfindungsgemäße Präkursor eine Verbindung der Formel III-a oder III-b, wobei R₁ aus der Gruppe ausgewählt ist, die Methyl, geschütztes und ungeschütztes Hydroxymethyl, Ethyl, n-Propyl, iso-Propyl, n-Butyl, iso-Butanyl, sec-Butyl, tert-Butyl, Phenyl, geschütztes und ungeschütztes Hydroxyphenyl, Benzyl und geschütztem und ungeschütztem Hydroxybenzyl umfasst.

Die als "Arylalkyl" bezeichneten chiralen Auxiliare stellen eine Arylgruppe dar, die eine oder mehrere C₁-C₆-Alkylgruppen trägt. Die Arylalkyl-Gruppe kann substituiert oder unsubstituiert sein. Beispiele sind - gegebenenfalls substituiertes - Benzyl, Phenylethyl und Arylglycinol, wobei die Aufzählung nicht abschließend ist.

Die als "Arylglycinol" bezeichneten chiralen Auxiliare stellen eine Untergruppe von als "Arylalkyl" bezeichneten chiralen Auxiliare dar. Beispielsweise können Präkursoren mit Arylglycinol-Gruppe eine Gruppe der Formel IV-a oder IV-b sein: 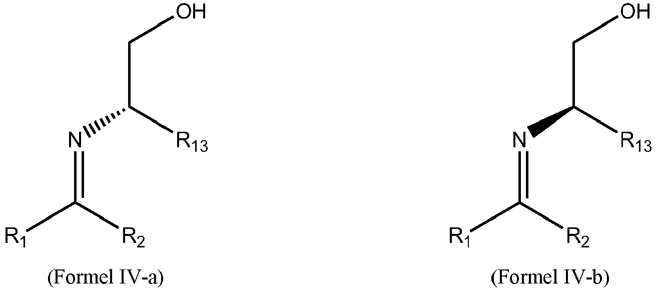 wobei R₁₃ substituiertes oder unsubstituiertes Aryl, wie oben definiert, ist. Beispiele einer Arylglycinol-Gruppe sind - gegebenenfalls substituiertes oder unsubstituiertes - Phenylglycinol, geschütztes oder ungeschütztes Phenylglycinol, wobei die Aufzählung nicht abschließend ist.

Die erfindungsgemäßen Präkursoren erlauben eine schnelle und diastereoselektive Umsetzung mit einem Kohlenstoff-11-Synthon. Diese Reaktion, die auch als Markierungsreaktion bezeichnet wird, ist diastereoselektiv. Aufgrund der anschließenden Abspaltung des chiralen Auxiliars, beispielsweise mittels Alkoholyse und/oder Hydrolyse, wird, entsteht ein Enantiomer im Überschuss. Das bedeutet, dass das erfindungsgemäße Verfahren im Ergebnis enantioselektiv ist.

Insbesondere können die erfindungsgemäßen Präkursoren verwendet werden, um Aminosäuren, insbesondere α-Aminosäuren und Derivate davon wie Aminosäure-Ester und Aminosäure-Amide herzustellen, deren Carbonyleinheit ein Kohlenstoff-11-Atom anstelle eines Kohlenstoff-12-Atoms aufweist. Die übrigen Kohlenstoffatome der Aminosäuren und Aminosäure-Derivate sind vorzugsweise keine Kohlenstoff-11-Atome. Die für die Synthese dieser Verbindungen benötigte Zeit liegt unter 20 min. Die radiochemische Ausbeute liegt über 10 % (nicht zerfallskorrigiert (n. d. c.)) bzw. über 20 % (zerfallskorrigiert (d.c.), jeweils bezogen auf gebildetes [¹¹C]HCN).

Die erfindungsgemäßen Präkursoren können beispielsweise durch Kondensation des entsprechenden Aldehyds mit einem Alkylsulfinyl-Amin in Gegenwart von wasserfreiem Kupfersulfat in Dichlormethan bei Raumtemperatur synthetisiert und optional anschließend chromatographisch gereinigt werden.

Nach Maßgabe der Erfindung ist ferner ein Verfahren zur diastereoselektiven Markierung eines erfindungsgemäßen Präkursors mit Kohlenstoff-11 vorgesehen, wobei der Präkursor mit einem Kohlenstoff-11-markierten Synthon zu einer Kohlenstoff-11 markierten Verbindung, die eine Kohlenstoff-11-markierte Carbonylgruppe aufweist, umgesetzt wird. Vorzugsweise ist das Kohlenstoff-11-markierte Synthon [¹¹C]R₃₁CN, wobei R₃₁ aus der Gruppe ausgewählt ist, die Wasserstoff, ein Alkalimetall wie Lithium, Natrium oder Kalium, Acetyl und Alkylsilyl umfasst. Vorzugsweise ist R₃₁ Wasserstoff, Natrium oder Kalium, besonders bevorzugt Wasserstoff oder ein Alkylsilyl, besonders bevorzugt Wasserstoff. Ein bevorzugtes Beispiel eines Alkylsilyls ist Trimethylsilyl. R₃₁ kann als Kation zu dem Anion [¹¹C]CN⁻ vorliegen. [¹¹C]R₃₁CN wird vorzugsweise als Gasstrom durch das Reaktionsgemisch, das den Präkursor und ggf. Lösungsmittel und Hilfsstoffe enthält, geführt. [¹¹C]R₃₁CN wird vorzugsweise durch Umwandlung aus [¹¹C]CO₂ hergestellt. Das kann mittels bekannter Verfahren geschehen. Die Umsetzung des Präkursors mit dem Synthon ist keine spezifische Reaktion. Vielmehr hängt es von dem chiralen Auxiliar und/oder den Reaktionsbedingungen, insbesondere dem oder den zugesetzten Hilfsstoffen und/oder dem oder den eingesetzten Lösungsmitteln, ab, welches der beiden Enantiomere bei der Umsetzung des Präkursors mit dem Synthon erhalten wird. Diese Umsetzung ist daher keine spezifische Reaktion.

Bei der Kohlenstoff-11 markierten Verbindung kann es sich beispielsweise um Aminosäuren und Derivate davon, wie Aminosäure-Ester und Aminosäure-Amide, handeln, bevorzugt α-Aminosäuren und Derivate davon, wie α-Aminosäure-Ester und α-Aminosäure-Amide, besonders bevorzugt L-α-Aminosäuren und Derivate davon, wie L-α-Aminosäure-Ester und L-α-Aminosäure-Amide. Bei der Kohlenstoff-11 markierten Verbindung kann es sich aber auch um D-α-Aminosäuren und Derivate davon, wie D-α-Aminosäure-Ester und D-α-Aminosäure-Amide, handeln. Das erfindungsgemäße Verfahren ermöglicht die enantioselektive Herstellung entweder der L-α-Aminosäuren und Derivaten davon, wie L-α-Aminosäure-Ester und L-α-Aminosäure-Amide, oder der D-α-Aminosäuren und Derivaten davon, wie D-α-Aminosäure-Ester und D-α-Aminosäure-Amide.

Beispielsweise kann es sich bei den Kohlenstoff-11 markierten Verbindung um eine Verbindung der Formel X handeln: 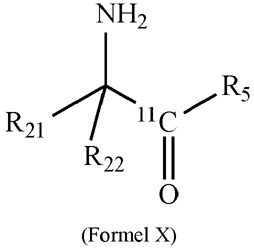 wobei
R₂₁ und R₂₂ die oben im Zusammenhang mit den Resten R₁ und R₂ des erfindungsgemäßen Präkursors angegebene Bedeutung haben,
R₅ aus der Gruppe ausgewählt ist, die OR₆ und NR₇R₈ umfasst, und
R₆, R₇ und R₈ unabhängig voneinander aus der Gruppe ausgewählt sind, die Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst. Die Ausdrücke "Alkyl", "Alkenyl", "Aryl", "Heteroaryl" und "substituiert" können die oben im Zusammenhang mit dem erfindungsgemäßen Präkursor angegebenen Bedeutungen haben. R₇ und R₈ sind vorzugsweise Wasserstoff.

Für die Herstellung einer Verbindung der Formel X, die bestimmte Reste R₂₁ und R₂₂ aufweisen soll, wird zweckmäßigerweise ein Präkursor ausgewählt, der - abgesehen von optional vorgesehen Schutzgruppen an den Resten R₁ und/oder R₂ - Reste R₁ und R₂ aufweist, die mit den Resten R₂₁ und R₂₂ der Verbindung der Formel X übereinstimmen. Wird ein Präkursor eingesetzt, der Schutzgruppen an den Resten R₁ und/oder R₂ aufweist, so kann das erfindungsgemäße Verfahren die Abspaltung der Schutzgruppen umfassen.

Beispiele für Verbindungen, der Formel X sind in der nachstehenden Tabelle 1 angegeben, wobei die Aufzählung nicht abschließend ist. Bei diesen Verbindungen ist R₂ jeweils Wasserstoff und R₅ Hydroxy.

**Tabelle 1**

| Formel-Nr. | Verbindung | R₁ |
|---|---|---|
| X-11 | 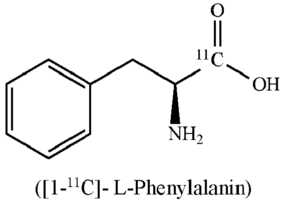 | 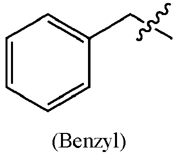 |
| X-12 | 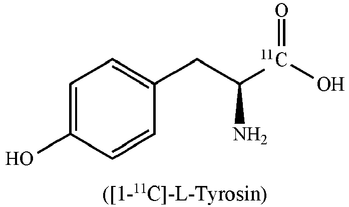 | 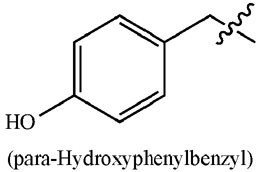 |
| X-13 | 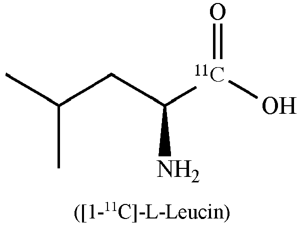 | 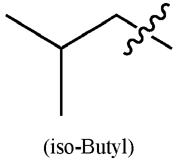 |
| X-14 | 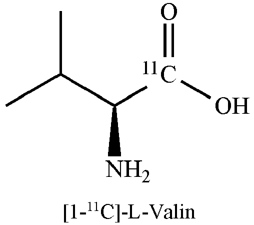 | 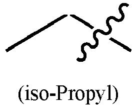 |
| X-15 | 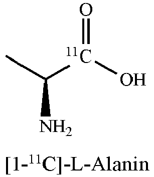 | 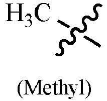 |
| X-16 | 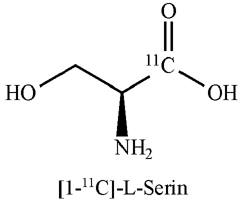 | 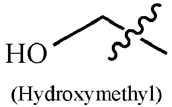 |

Das erfindungsgemäße Verfahren kann die Umsetzung des erfindungsgemäßen Präkursors mit dem Kohlenstoff-11-markierten Synthon zu einer Verbindung der Formel V umfassen: 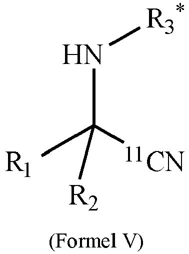 wobei R₁, R₂ und R₃ die in Anspruch 1 angegebenen Bedeutungen aufweisen. Die Reaktionsbedingungen werden bevorzugt so gewählt, dass das Kohlenstoff-11-markierte Synthon durch nukleophile Addition an den Präkursor anbindet. Die Umsetzung erfolgt vorzugsweise in einem aprotischen Lösungsmittel, beispielsweise 1,4-Dioxan, Tetrahydrofuran, Diethylether, Methyl-tert-Butylether, Toluol, Benzol, Dichlormethan und andere halogenierte Lösungsmittel, wobei die Aufzählung nicht abschließend ist, einem protischen organischen Lösungsmittel, beispielsweise Methanol, Ethanol, iso-Proyplalkohol, n-Propylalkohol, n-Butanol, oder den Lösungsmitteln Wasser, DMSO und DMF, wobei die Aufzählung nicht abschließend ist, oder Gcmischcn aus einem aprotischen Lösungsmittel und einem protischen organischen Lösungsmittel. Bevorzugt ist ein Lösungsmittelgemisch, das ein Mischungsverhältnis des aprotischen Lösungsmittels und dem protischen organischen Lösungsmittel in jedem denkbaren Verhältnis aufweist. Besonders bevorzugt ist ein Mischungsverhältnis von 8 zu 2, bezogen auf das Volumen. Ein besonders bevorzugtes Lösungsmittelgemisch ist ein Gemisch aus 1,4-Dioxan und Methanol. Die Umsetzung wird vorzugsweise bei einer Temperatur von 10 bis 60 °C, besonders bevorzugt bei Raumtemperatur durchgeführt. Die Reaktionszeit kann zwischen 1 und 10 min liegen. Das Reaktionsgemisch kann neben dem Präkursor, dem Kohlenstoff-11-markierten Synthon und dem Lösungsmittel einen oder mehrere Hilfsstoffe enthalten. Bei einem solchen Hilfsstoff kann es sich um ein Additivsalz wie Cäsiumfluorid, "Tetrabutylammoniumfluorid" (TBAF), Zinkdiiodid, Aluminiumtrichlorid oder andere Lewissäuren oder Fluoridsalze handeln, wobei Cäsiumfluorid bevorzugt ist. Wird [¹¹C]HCN als Synthon eingesetzt, so liegen die Markierungsausbeuten bei 70 bis 95 %, bezogen auf das eingesetzte [¹¹C]HCN.

Vorzugsweise wird eine Verbindung der Formel I-b zu einer Verbindung der Formel V-a umgesetzt (Schema la):

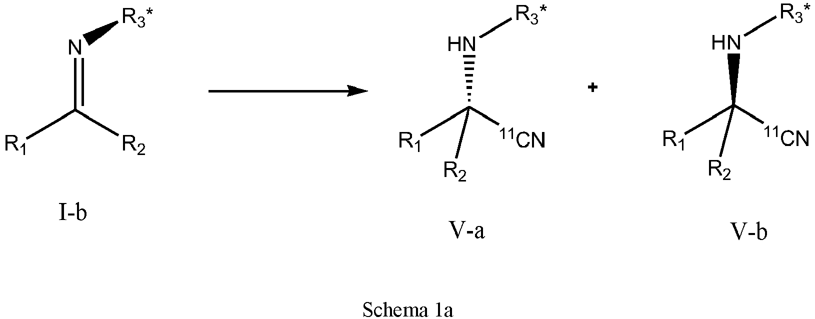

Die Umsetzung gemäß Schema 2a ist insbesondere dann vorteilhaft, wenn das erfindungsgemäße Verfahren zur Herstellung von L-Aminosäuren und deren Derivaten eingesetzt werden soll. Es hängt vom dem Präkursor, insbesondere dem eingesetzen chiralen Auxiliar des Präkursors, und den Reaktionsbedinungen, insbesondere dem oder den Hilfsstoffen und dem oder den Lösungsmitteln ab, welche der beiden Verbindungen V-a oder V-b im Überschuss erhalten wird. Alternativ kann eine Verbindung der Formel I-a zu einer Verbindung der Formel Va oder V-b umgesetzt werden (Schema 1b). Es hängt vom dem Präkursor, insbesondere dem eingesetzten chiralen Auxiliar des Präkursors, und den Reaktionsbedingungen, insbesondere dem oder den Hilfsstoffen und/oder dem oder den eingesetzten Lösungsmitteln ab, welche der beiden Verbindungen V-a oder V-b im Überschuss erhalten wird. Die Umsetzung des Präkursors I mit dem Synthon ist keine spezifische Reaktion.

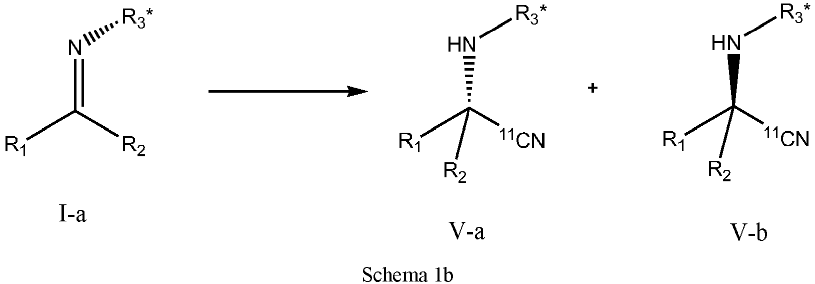

Im nächsten Schritt kann die Verbindung der Formel V mittels Alkoholyse, die zu oder Hydrolyse (Alkoholyse führt zu OR6, Hydrolyse führt zu NR7R8 oder OH) in eine Verbindung der Formel XI überführt werden, wobei die Alkoholyse zu R₅ = OR₆ führt und die Hydrolyse zu R₅ = NR₇R₈ oder OH führt: 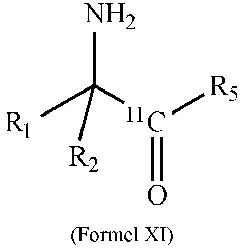 wobei R₁, R₂ und R₅ wie oben definiert sind, mit der Maßgabe, dass R₅ nicht OH ist (d. h. R₅ ist nicht Wasserstoff). Die Verbindung der Formel XI umfasst keine Aminosäuren. Abgesehen davon und sofern der Präkursor keine Schutzgruppe aufweist, entspricht die Verbindung der Formel XI der Verbindung der Formel X. Das heißt R₁ und R₂ entsprechen R₂₁ und R₂₂. Weist der Präkursor hingegen Schutzgruppen auf, so können die R₁ und/oder R₂, je nach dem, ob beide Reste eine Schutzgruppe aufweisen oder nur einer davon, durch Abspalten der Schutzgruppen in die Reste R₂₁ und R₂₂ überführt werden.

Die Überführung der Verbindung der Formel V-a in eine Verbindung der Formel XIa ist in Schema 2a, die Überführung der Verbindung der Formel V-b in eine Verbindung der Formel XIb ist in Schema 2b gezeigt:

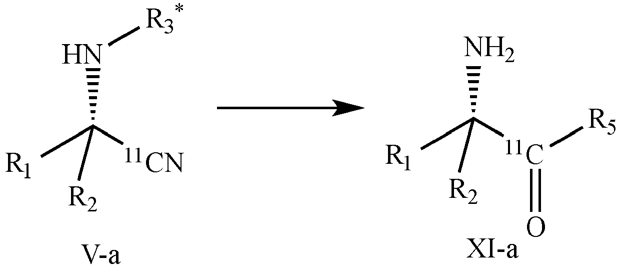

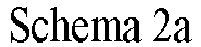

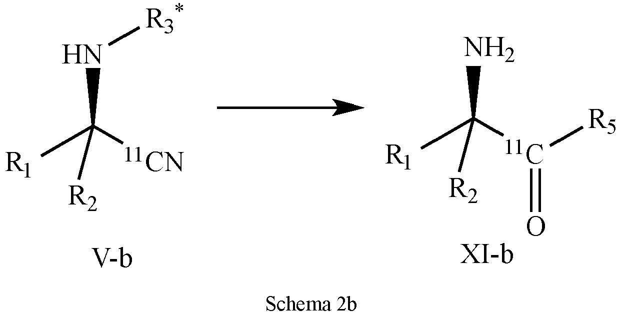

Die Umsetzung gemaß Schema 2a ist insbesondere dann vorteilhaft, wenn das erfindungsgemäße Verfahren zur Herstellung von L-Aminosäuren und deren Derivaten eingesetzt werden soll.

Anschließend kann die Verbindung der Formel XI mittels Hydrolyse in eine Verbindung der Formel XII überführt werden: 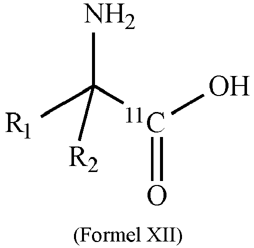 wobei R₁ und R₂ oben definiert sind. Die Verbindung der Formel XII umfasst nur Aminosäuren. Abgesehen davon und sofern der Präkursor keine Schutzgruppe aufweist, entspricht die Verbindung der Formel XII der Verbindung der Formel X. Das heißt R₁ und R₂ entsprechen R₂₁ und R₂₂. Weist der Präkursor hingegen Schutzgruppen auf, so können die R₁ und/oder R₂, je nach dem, ob beide Reste eine Schutzgruppe aufweisen oder nur einer davon, durch Abspalten der Schutzgruppen in die Reste R₂₁ und R₂₂ überführt werden. Die Hydrolyse kann unter dem Fachmann bekannten Reaktionsbedingungen durchgeführt werden. Die Hydrolyse kann unter sauren oder basischen Bedingungen durchgeführt werden.

Die Überführung der Verbindung der Formel XI-a mittels Hydrolyse in eine Verbindung der Formel XII-a ist in Schema 3a, die Überführung der Verbindung der Formel XI-b mittels Hydrolyse in eine Verbindung der Formel XII-b ist in Schema 3b gezeigt:

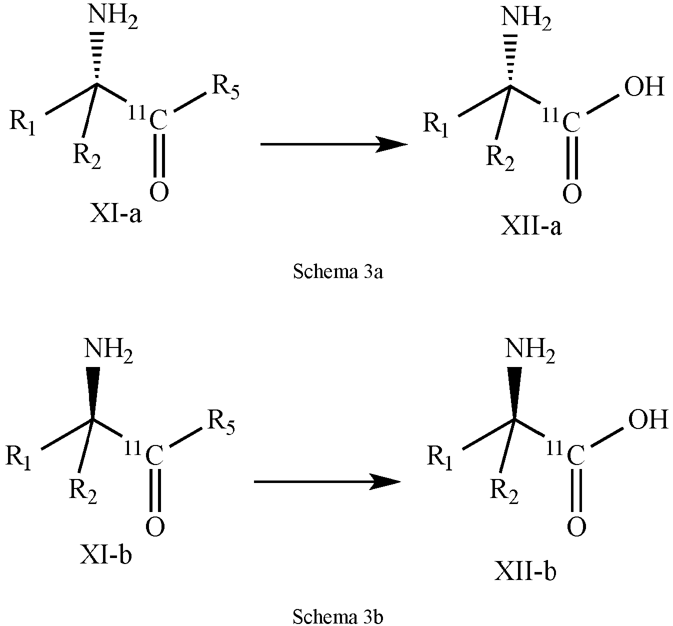

Die Umsetzung gemäß Schema 3a ist insbesondere dann vorteilhaft, wenn das erfindungsgemäße Verfahren zur Herstellung von L-Aminosäuren und deren Derivaten eingesetzt werden soll.

Anstelle einer Alkoholyse der Verbindung der Formel V zu einer Verbindung der Formel XI und der optional anschließend Hydrolyse der Verbindung der Formel XI zu einer Verbindung der Formel XII kann eine direkte Hydrolyse der Formel V zu einer Verbindung der Formel XII vorgesehen sein.

In einer bevorzugten Ausführungsform des erfindungsgemäßen Verfahrens wird ein Präkursor der Formel II 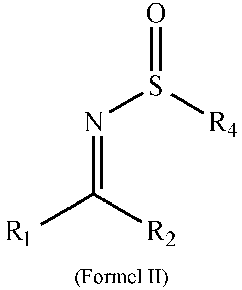 zur Herstellung einer Verbindung der Formel XIII: 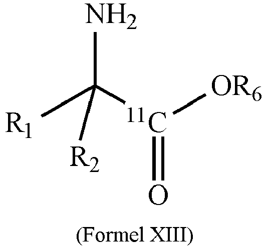 verwendet, wobei R₁, R₂, R₄ und R₆ die oben angegebenen Bedeutungen haben. Sofern die Reste R₁ und/oder R₂ keine Schutzgruppen aufweisen, kann Verbindung XIII Verbindung X entsprechen, wobei R₅ -O-R₆ ist. R₄ ist vorzugsweise tert.-Butyl, R₂ vorzugsweise Wasserstoff. Weisen die Reste R₁ und/oder R₂ Schutzgruppen auf, so kann die Verbindung der Formel XIII durch Abspaltung der Schutzgruppen in eine Verbindung der Formel X überführt werden.

Die bevorzugte Ausführungsform kann zunächst das Umsetzen der Verbindung der Formel II mit dem Kohlenstoff-11-markierten Synthon zu einer Verbindung der Formel XIV umfassen: 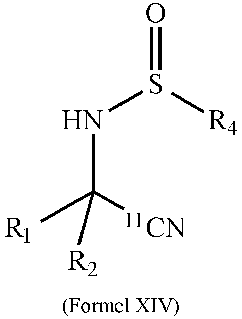 wobei R₁, R₂ und R₄ oben angegebenen Bedeutungen haben. Die Reaktionsbedingungen werden bevorzugt so gewählt, dass das Kohlenstoff-11-markierte Synthon durch nukleophile Addition an den Präkursor anbindet. Die Umsetzung erfolgt vorzugsweise in einem aprotischen Lösungsmittel, beispielsweise 1,4-Dioxan, Tetrahydrofuran, Diethylether, Methyl-tert-Butylether, Toluol, Benzol, Dichlormethan und andere halogenierte Lösungsmittel, wobei die (Aufzählung nicht abschließend ist), einem protischen organischen Lösungsmittel, beispielsweise Methanol, Ethanol, iso-Proyplalkohol, n-Propylalkohol, n-Butanol, oder den Lösungsmitteln Wasser, DMSO und DMF, wobei die Aufzählung nicht abschließend ist, oder Gemischen aus einem aprotischen Lösungsmittel und einem protischen organischen Lösungsmittel. Bevorzugt ist ein Lösungsmittelgemisch, das ein Mischungsverhältnis des aprotischen Lösungsmittels und dem protischen organischen Lösungsmittel in jedem erdenklichen Verhältnis, vorzugsweise in einem Bereich von 7 zu 3 bis 9 zu 1, bezogen auf das Volumen, aufweist. Besonders bevorzugt ist ein Mischungsverhältnis von 8 zu 2, bezogen auf das Volumen. Ein besonders bevorzugtes Lösungsmittelgemisch ist ein Gemisch aus 1,4-Dioxan und Methanol. Die Umsetzung wird vorzugsweise bei einer Temperatur von 10 bis 60 °C, besonders bevorzugt bei Raumtemperatur durchgeführt. Die Reaktionszeit kann zwischen 1 und 10 min liegen. Das Reaktionsgemisch kann neben dem Präkursor, dem Kohlenstoff-11-markierten Synthon und dem Lösungsmittel einen oder mehrere Hilfsstoffe enthalten. Beispiele eines solchen Hilfsstoffes sind Cäsiumfluorid, Tetrabutylammoniumfluorid, Zinkdiiodid, Aluminiumtrichlorid oder andere Lewissäuren oder Fluoridsalze. Wird [¹¹C]HCN als Synthon eingesetzt, so liegen die Markierungsraten bei 70 bis 95 %, bezogen auf das eingesetzte [¹¹C]HCN. Die Überführung der Verbindung der Formel II-a in ein Gemisch der Enantiomere der Formel XIV-a-1 und XIV-a-2 ist in Schema 4a, die Überführung der Verbindung der Formel II-b in ein Gemisch der Enantiomere der Formel XIV-b-1 und XIV-b-2 ist in Schema 4b gezeigt. Das chirale Auxiliar des Präkursors und die gewählten Reaktionsbedingungen, insbesondere der oder die eingesetzten Hilfsstoffe und/oder das oder die eingesetzten Lösungsmittel bestimmen, welche der beiden Reaktionsprodukte im Überschuss erhalten wird.

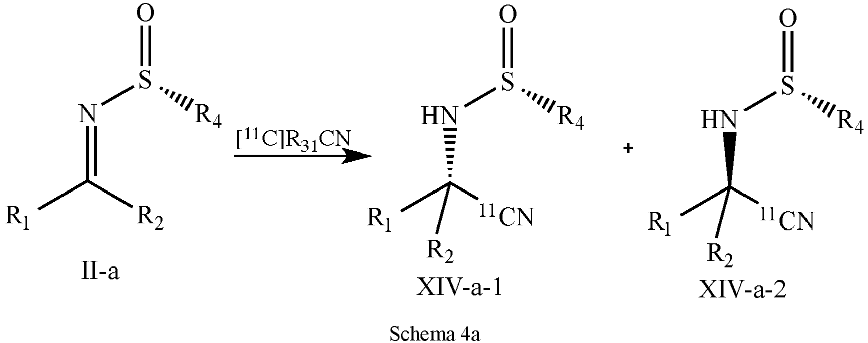

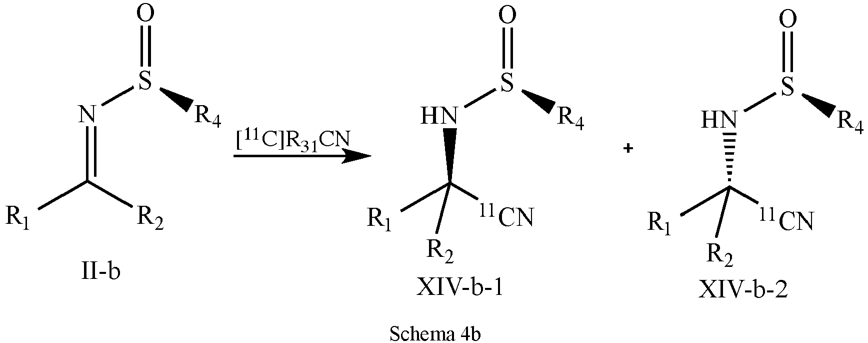

Die Umsetzung gemaß Schema 4b ist insbesondere dann vorteilhaft, wenn das erfindungsgemäße Verfahren zur Herstellung von L-Aminosäuren und deren Derivaten eingesetzt werden soll.

Die Verbindung der Formel IV kann anschließend mittels Alkoholyse in eine Verbindung der Formel XV überführt werden: 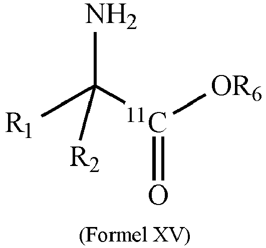 wobei R₁, R₂ und R₆ wie oben definiert sind, mit der Maßgabe, dass R₆ nicht Wasserstoff ist. Die Verbindung der Formel XV umfasst keine Aminosäuren. Abgesehen davon und sofern der Präkursor keine Schutzgruppe aufweist, entspricht die Verbindung der Formel XV der Verbindung der Formel X. Das heißt R₁ und R₂ entsprechen R₂₁ und R₂₂. Weist der Präkursor hingegen Schutzgruppen auf, so können die R₁ und/oder R₂, je nach dem, ob beide Reste eine Schutzgruppe aufweisen oder nur einer davon, durch Abspalten der Schutzgruppen in die Reste R₂₁ und R₂₂ überführt werden.

Die Alkoholyse wird unter Einsatz eines Alkohols durchgeführt, der die Gruppe R₆ aufweist und in den Schemen 5a und 5b als R₆OH dargestellt ist. Die Alkoholyse kann unter dem Fachmann bekannten Reaktionsbedingungen durchgeführt werden. Die Reaktionszeit kann beispielsweise 5 bis 60 Minuten, die Reaktionstemperatur beispielsweise 60 bis 150 °C betragen. Die Überführung der Verbindung der Formel XIV-a-1 in eine Verbindung der Formel XV-a ist in Schema 5a-1, die Überführung der Verbindung der Formel XIV-a-2 in eine Verbindung der Formel XVb ist in Schema 5a-2, die Überführung der Verbindung der Formel XIV-b-1 in eine Verbindung der Formel XV-b ist in Schema 5b-1 und die Überführung der Verbindung der Formel XIV-b-2 in eine Verbindung der Formel XV-a ist in Schema 5b-2 gezeigt.

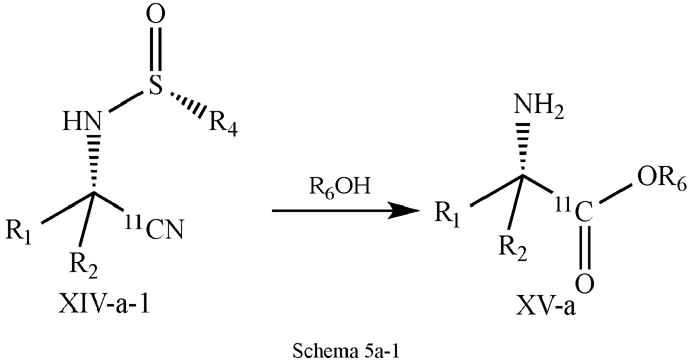

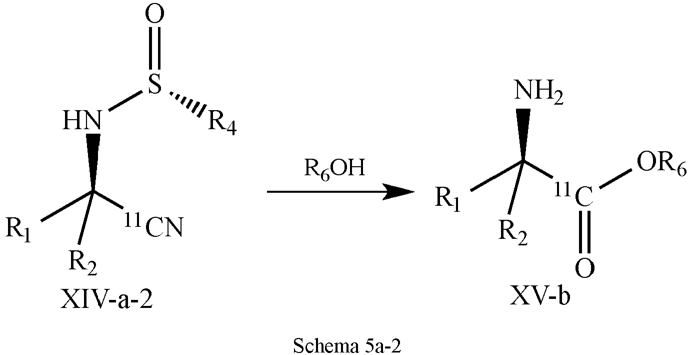

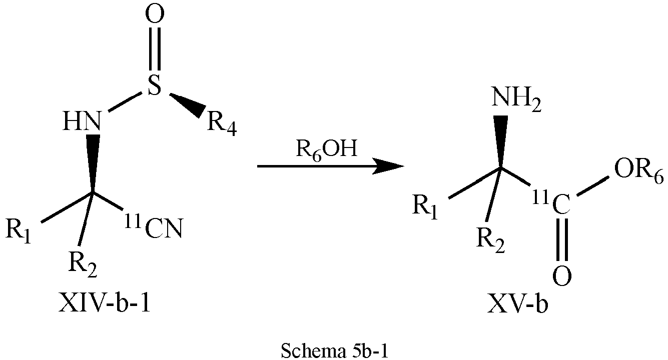

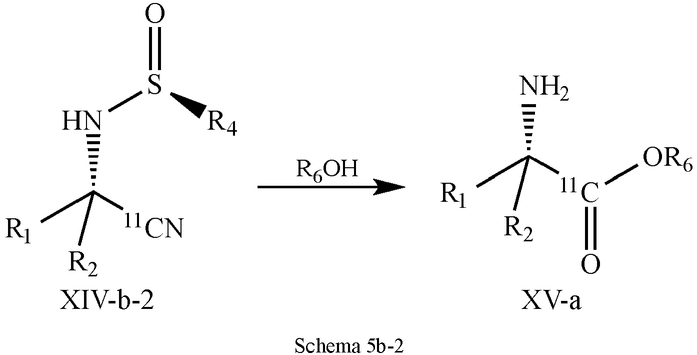

Für die Herstellung von L-Aminosäuren und ihren Derivaten wird vorzugsweise die Verbindung der Formel XV-a hergestellt.

Die Verbindung der Formel XV kann anschließend mittels Hydrolyse in eine Verbindung der Formel XII überführt werden: 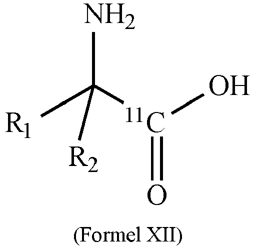 wobei R₁ und R₂ wie oben definiert sind.. Sofern der Präkursor keine Schutzgruppe aufweist, entspricht die Verbindung der Formel XII der Verbindung der Formel X, wobei dass R₅ Hydroxy ist. Das heißt R₁ und R₂ entsprechen R₂₁ und R₂₂. Weist der Präkursor hingegen Schutzgruppen auf, so können die R₁ und/oder R₂, je nach dem, ob beide Reste eine Schutzgruppe aufweisen oder nur einer davon, durch Abspalten der Schutzgruppen in die Reste R₂₁ und R₂₂ überführt werden.

Die Hydrolyse kann unter sauren Bedingungen durchgeführt werden. Beispielsweise kann wässerige konzentrierte Salzsäure zur Hydrolyse eingesetzt werden. Die Reaktionstemperatur kann beispielsweise bei 100 bis 250 °C liegen, die Reaktionszeit bei 2 bis 20 min. Die Überführung der Verbindung der Formel XV-a mittels Hydrolyse in eine Verbindung der Formel XII-a ist in Schema 6a, die Überführung der Verbindung der Formel XV-b mittels Hydrolyse in eine Verbindung der Formel XII-b ist in Schema 6b gezeigt:

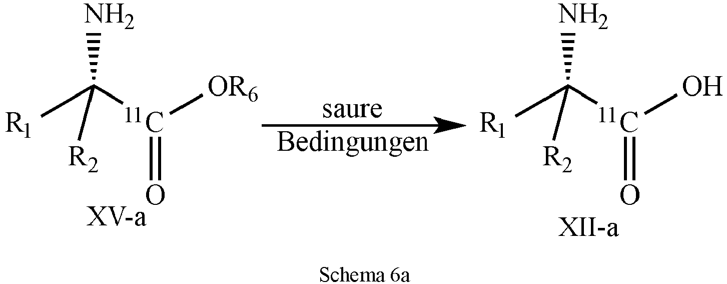

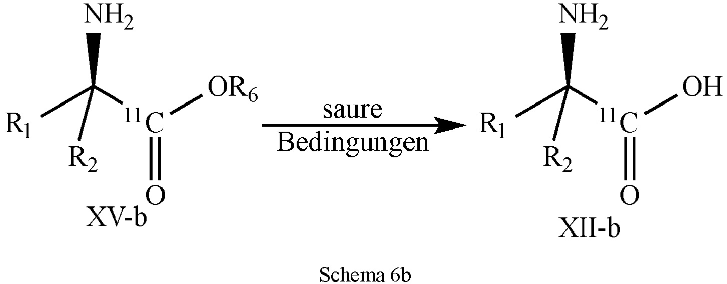

Für die Herstellung von L-Aminosäuren und ihren Derivaten wird die Verbindung der Formel XV-a zur Verbindung der Formal XII-a umgesetzt. Für die Herstellung von D-Aminosäuren und ihren Derivaten wird die Verbindung der Formel XV-b zur Verbindung der Formal XII-b umgesetzt.

Alternativ zu einer Alkoholyse der Verbindungen der Formel XIV, an die sich optional eine Hydrolyse anschließen kann, können die Verbindungen der Formel XIV direkt einer direkten Hydrolyse unterzogen werden. Dabei wird die Verbindung der Formel XIV zunächst in eine Verbindung der Formel XVI überführt 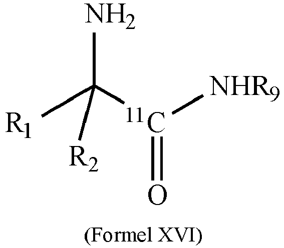 wobei R₁ und R₂ wie in Anspruch 6 definiert sind und R₉ aus der Gruppe ausgewählt ist, die Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst. R₉ ist vorzugsweise Wasserstoff. Die Hydrolyse kann dann fortgesetzt werden, indem die Verbindung der Formel XVI in eine Verbindung der Formel XII überführt wird: 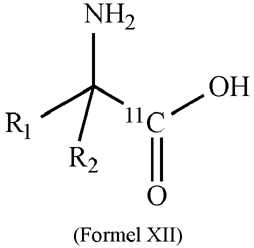 wobei R₁ und R₂ wie oben definiert sind. Die direkte Hydrolyse kann unter sauren oder basischen Bedingungen, vorzugsweise unter sauren Bedingungen durchgeführt werden. Beispielsweise kann wässrige konzentrierte Salzsäure zur sauren Hydrolyse eingesetzt werden. Die Reaktionstemperatur kann beispielsweise bei 100 bis 250 °C liegen, die Reaktionszeit bei 2 bis 20 min.

Die Überführung der Verbindung der Formel XIV-a-1 in eine Verbindung der Formel XVI-a ist in Schema 7a-1, die Überführung der Verbindung der Formel XIV-a-2 in eine Verbindung der Formel XVI-b ist in Schema 7a-2, die Überführung einer Verbindung der Formel XIV-b-1 in eine Verbindung der Formel XVI-b ist in Schema 7b-1 und die Überführung einer Verbindung der Formel XIV-b-2 in eine Verbindung der Formel XVI-a ist in Schema 7b-2 gezeigt:

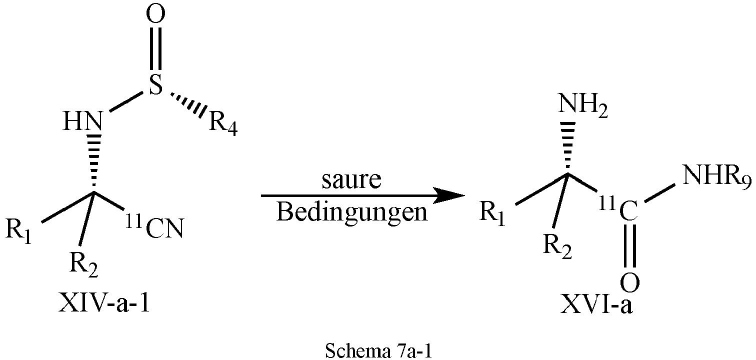

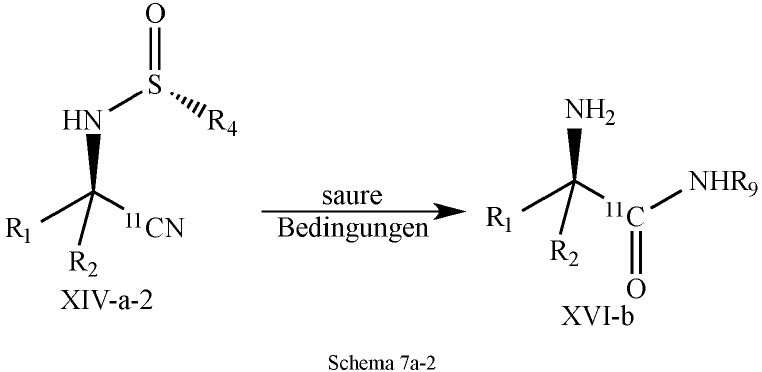

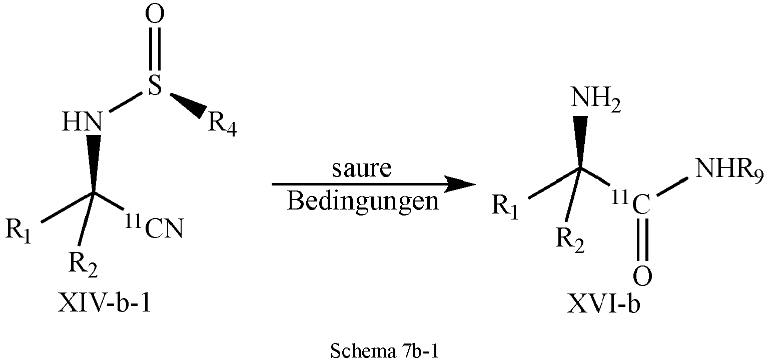

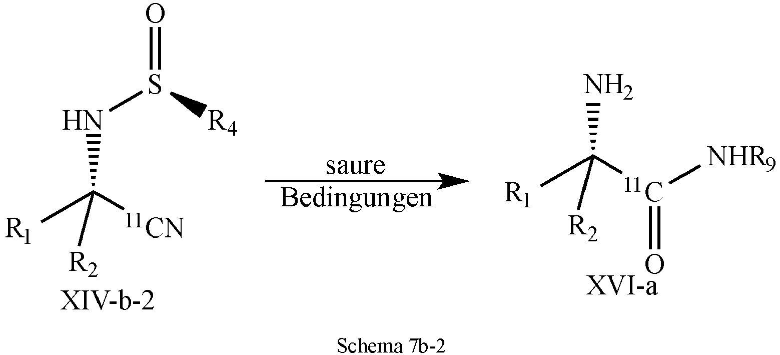

Die Überführung der Verbindung der Formel XVI-a mittels Hydrolyse in eine Verbindung der Formel XII-a ist in Schema 8a, die Überführung der Verbindung der Formel XVI-b mittels Hydrolyse in eine Verbindung der Formel XII-b ist in Schema 8b gezeigt:

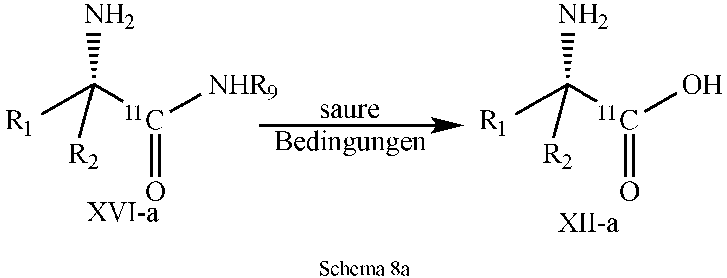

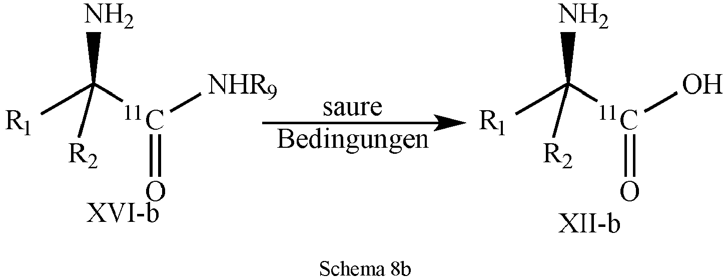

Für die Herstellung von L-Aminosäuren und ihren Derivaten wird die Verbindung der Formel XVI-a zur Verbindung der Formal XII-a umgesetzt. Für die Herstellung von D-Aminosäuren und ihren Derivaten wird die Verbindung der Formel XVI-b zur Verbindung der Formal XII-b umgesetzt.

Sind die Reste R₁ und R₂ ungeschützte Reste so kann vorgesehen sein, dass die Reste R₁ und R₂ einer Verbindung der Formeln XI, XII, XIII, XV oder XVI den Resten R₂₁ und/oder R₂₂ der Verbindung X entsprechen. Ist hingegen der Rest R₁ ein geschützter Rest, so kann vorgesehen sein, dass der Rest R₁ einer Verbindung der Formeln XI, XII, XIII, XV oder XVI in den Rest R₂₁ der Verbindung X durch Abspalten einer oder mehrerer Schutzgruppen überführt wird. Ist Rest R₂ ein geschützter Rest, so kann vorgesehen sein, dass der Rest R₂ einer Verbindung der Formeln XI, XII, XIII, XV oder XVI in den Rest R₂₂ der Verbindung X durch Abspalten einer oder mehrerer Schutzgruppen überführt wird.

Das erfindungsgemäße Verfahren kann in einem Mikrofluidik-System, insbesondere einem Mikrofluidik-Reaktor durchgeführt werden. Bei dem Mikrofluidik-System kann es sich beispielsweise um die von General Electric angebotene ISAR®-Plattform handeln. Das Mikrofluidik-System kann Reinigungskartuschen umfassen, um die Zielverbindung, d. h. eine Verbindung der Formel X, oder eine Zwischenstufe einer Reinigung zu unterziehen. Das Mikrofluidik-System kann einen Polymerchip aufweisen, auf dem die Synthese der Kohlenstoff-11-markierten Aminosäuren oder Derivate davon durchgeführt werden kann. Das erfindungsgemäße Verfahren kann ein automatisiertes Verfahren sein.

Das erfindungsgemäße Verfahren wird vorzugsweise als nicht-trägergebundene Synthese (n.c.a-Synthese) durchgeführt.

Zum Erhalt einer enantiomerenreinen Verbindung der Formel X kann das erfindungsgemäße Verfahren einen Reinigungsschritt zur Abtrennung des ungewünschten Enantiomers umfassen. Der Reinigungsschritt kann beispielsweise eine enzymatische Reinigung oder eine chirale Trennung sein. Ist das Ziel des erfindungsgemäßen Verfahrens die Herstellung einer L-Aminosäure oder eines Derivates davon, so kann mittels des Reinigungsschrittes die entsprechende D-Aminosäure bzw. ihr Derivat abgetrennt werden.

Das erfindungsgemäße Verfahren ermöglicht die Synthesezeiten von weniger als 20 min für die Herstellung von Kohlenstoff-11-markierten Aminosäuren oder ihren Derivaten, und zwar einschließlich (i) der Umwandung von [¹¹C]CO₂ in [¹¹C]R₃₁CN, (ii) dem Einbau von [¹¹C]R₃₁CN in den Präkursor und (iii) die Alkoholyse, und optional anschließende Hydrolyse, oder die direkte Hydrolyse der Nitrilgruppe in die ggf. substituierte Carbonsäure.

Die mittels des erfindungsgemäßen Verfahrens hergestellten Kohlenstoff-11-markierten Aminosäuren oder Derivate davon können weiter zu Peptiden, insbesondere Oligopeptiden, oder Peptidanaloga umgesetzt werden. Die mittels des erfindungsgemäßen Verfahrens hergestellten Kohlenstoff-11-markierten Aminosäuren oder Derivate davon können zum Einbau in Peptide, Proteine oder Antikörper verwendet werden.

Die Erfindung beruht, ohne an eine Erklärung gebunden sein zu wollen, auf folgenden Erkenntnissen: Durch gezielte Wahl des optimalen Lösungsmittels, vorzugsweise 1,4-Dioxan in einem Verhältnis zu Methanol von 4 : 1, und des Hilfsstoffes, vorzugsweise Cäsiumfluorid (CsF), können hohe Diastereomerenüberschüsse (de) in sehr kurzen Reaktionszeiten erreicht werden. Zum Einen sind in reinem Dioxan, Dichlormethan, Toluol oder anderen aprotisch unpolaren Lösungsmitteln unter Zusatz von Cäsiumfluorid zwar die de-Werte sehr gut (70% bis > 90%). Allerdings ist CsF in diesen Lösungsmitteln nur schlecht löslich, was dazu führt, dass die Reaktionszeit inakzeptabel auf mehrere Stunden bzw. Tage verlängert wird. Zum Anderen ist der Hilfsstoff CsF in protisch polaren Lösungsmitteln wie Wasser, DMSO oder verschiedenen Alkoholen wie Methanol, Ethanol und Propanol zwar besser löslich. Somit verkürzen sich die Reaktionszeiten auf unter eine Stunde. Allerdings verändert sich die Stereoselektivität zum schlechteren (< 50 %) und dreht sich dabei sogar um. Diese beiden gegenläufigen Effekte haben zu der Erkenntnis geführt, dass es prinzipiell möglich ist, durch geschickte Wahl des oder der Lösungsmittel und/oder des oder der Hilfsstoffe aus dem selben enantiomerenreinen (S)-Sulfinylimin-Präkursor sowohl die L-Aminosäure als auch die D-Aminosäure in guter Stereoselektivität (> 30 % ee bzw. de bei der Zwischenverbindung) darzustellen. Die Erfinder haben festgestellt, dass in einem Gemisch aus Dioxan mit wenig Methanol oder Wasser, d. h. einem Anteil von vorzugsweise 10 bis 20 %, bezogen auf das Volumen, an dem Lösungsmittelgemisch die Löslichkeit des Hilfsstoffes CsF stark steigt und somit die Reaktionszeit auf weniger als 20 min sinkt. Dabei lagen die de-Werte bei guten 70 bis 80 %. Nach der Freisetzung der angereicherten Aminosäure oder ihrem Derivat durch saure Hydrolyse, wobei auch eine basische Hydrolyse möglich ist, kann eine enzymatische Reinigung erfolgen. Beispielsweise kann durch immobilisierte D-α-Aminosäure-Oxidase (D-AAO) die D-Aminosäure entfernt werden. Dies ist auch umgekehrt möglich. Durch Verwendung des Enzyms L-α-Aminosäure-Oxidase (L-AAO) ist prinzipiell auch die Darstellung der enantiomeren D-Aminosäure möglich.

Mittels des erfindungsgemäßen Verfahrens können Aminosäuren und Derivate davon - ohne Reinigung wie eine enzymatische oder chirale Reinigung - mit einem *ee*-Wert von 30 % oder höher, bevorzugt 50 % oder höher, stärker bevorzugt 70 % oder höher und besonders bevorzugt 90 % oder höher erhalten werden. Mittels des erfindungsgemäßen Verfahrens können spezifische Aktivitäten von ca. 20 bis 70 GBq/µmol, erhalten werden. Es ist möglich, höhere spezifische Aktivitäten zu erreichen, was u. a. den Einsatz äußerst sauberer Gase, insbesondere Stickstoff und/oder Wasserstoff erfordert.

Die Vorteile des erfindungsgemäßen Verfahrens gegenüber dem Verfahren von Xing et al. liegen insbesondere in der diastereoselektiven Umsetzung eines chiralen Präkursors mit [11C]R₃₁CN. Es können gezielt L- oder D- Aminosäuren hergestellt werden. Zudem muss der Präkursor nicht erst im Synthesemodul intermediär hergestellt werden, sondern ist thermodynamisch stabil und somit lagerbar.

Nach Maßgabe der Erfindung kann ferner ein Reagenzienkit zur vorgesehen sein, das einen erfindungsgemäßen Präkursor für die enantioselektive Herstellung einer Kohlenstoff-11-markierten Aminosäure oder eines Derivates davon, Lösungsmittel und einen oder mehrere Hilfsstoffe umfassen kann. Zusätzlich kann das erfindungsgemäße Reagenzienkit eine oder mehrere Reinigungskartuschen umfassen. Das erfindungsgemäße Reagenzienkit ermöglicht die automatisierte enantioselektive Herstellung der Kohlenstoff-11-markierten Aminosäure oder des Derivates davon.

Die Erfindung wird nachstehend anhand von Ausführungsbeispielen, die die Erfindung nicht einschränken sollen, näher erläutert. Dabei zeigen
- Fig. 1: ein radiochemisches HPLC-Chromatogramm, das die Ergebnisse der Markierungsreaktion bei der diastereoselektiven Herstellung von [1-¹¹C]-L-Tyrosin (Verbindung X-12) zeigt;
- Fig. 2: ein radiochemisches HPLC-Chromatogramm, das die Reaktionsprodukte nach der Hydrolyse und vor der Aufreinigung zeigt;
- Fig. 3: ein radiochemisches HPLC-Chromatogramm, das die Ergebnisse der Aufreinigung mit einer SPEKartusche zeigt; und
- Fig. 4: ein radiochemisches HPLC-Chromatogramm, das die Ergebniise der enzymatischen Aufreinigung zeigt.

### Allgemeine Synthesevorschrift für die diastereoselektive Markierung von α-Aminosäuren mit [¹¹C]HCN

### Start: Bildung von [¹¹C]HCN aus [¹¹C]CO₂

Zunächst wird das im Zyklotron gebildete [¹¹C]CO₂ in die Heißzelle geleitet und dort auf einem Molsieb (4Ä) absorbiert. Währenddessen strömt Stickstoff durch das Molsieb und zieht ungewollte Verunreinigungen mit sich. Das Molsieb wird nun auf eine Temperatur von 350 °C erhitzt, wobei ab ca. 200 °C das abgefangene [¹¹C]CO₂ vom Molsieb desorbiert wird und durch den Stickstoffstrom (von 40 mL/min) weitergeleitet wird. Im nächsten Reaktionsschritt wird Wasserstoffgas (20 ml/min) zu dem Stickstoff/[¹¹C]CO₂-Gemisch geleitet und dieses Gasgemisch wird über einen 400 °C heißen Nickelkatalysator geleitet. Bei diesem Prozess wird das [¹¹C]CO₂ zu ¹¹C-Methan ([¹¹C]CH₄) und Wasser reduziert. Wasser und nicht reagiertes [¹¹C]CO₂ werden abgefangen (mit Ascarit und Siccapent) und zum übrig bleibenden Methanstrom wird gasförmiges Ammoniak (5 ml/min) zugegeben. Dieses Gasgemisch wird über einen Platindraht bei 950 °C geleitet und dabei entsteht [¹¹C]HCN. Dieser Gasstrom wird nun direkt für die Synthese von Aminosäuren verwendet.

### A) Markierungs-Reaktion

In ein kleines Reaktorvial (V=1-3 mL) werden ein Präkursors der Formel II, vorzugsweise der Formel III (zwischen 0,5 -5 mg) und Cäsiumfluorid (1-2 eq), gelöst in ca 200-800 µL eines Lösungsgemisches, bestehend aus 1,4-Dioxan und Methanol (Mischungsverhältnis 7 : 3 bis 9 : 1, vorzugsweise 8 : 2, bezogen auf das Volumen), vorgelegt. Das radiomarkierte, gasförmige [¹¹C]HCN wird mit einem Trägergasstrom (Mix aus N₂, H₂ und NH₃) von ca. 50-500 mL/min in dieses Reaktorvial eingeleitet. Die Reaktion wird bei Raumtemperatur durchgeführt, aber auch höhere und tiefere Temperaturen sind möglich, z. B. 10 bis 60 °C.

Die vollständige Bildung von [¹¹C]HCN dauert, je nach eingestelltem Gasfluss zwischen 1 und 10 Minuten. Nach Beendigung der [¹¹C]HCN-Bildungsreaktion wird noch kurz (0,1-5 min) Wasserstoffgas (ca. 20-150 ml/min) durch das Reaktorvial geleitet um überschüssiges Ammoniak zu entfernen. Auf diese Weise entsteht das Aminosäurenitril der Formel II, vorzugsweise der Formel V als Zwischenverbindung. Die Markierungsraten liegen üblicherweise zwischen 70-95 % des eingesetzten [¹¹C]HCNs.

### B) Automatisierte Hydrolyse

Die Reaktionslösung wird nach beendeter Markierungsreaktion in ein weiteres Reaktorvial (V = 2 bis 4 ml) überführt. (Das ist aber nicht erforderlich. Vielmehr kann die Hydrolyse auch im selben Reaktorvial durchgeführt werden). Das Reaktorvial wird von außen erhitzt und Wasserstoffgas wird durch die Reaktionslösung durchgeleitet, wodurch die Lösung etwas eingeengt wird. Als nächstes wird konzentrierte HCl (400-1000 µl) in dieses Reaktorvial gegeben und die Reaktionslösung für 2-20 min bei 100-250 °C erhitzt. Nach beendeter Hydrolyse wird die Lösung mit soviel an Puffer (K₃PO₄) versetzt, so dass der pH-Wert der Lösung auf pH = 2-2,5 eingestellt wird. Vorteilhafterweise liegt der pH-Wert ca. 2 Punkte unter dem isoelektischen Punkt der Zielverbindung, d. h. einer α-Aminosäure der Formel X.

### C) Aufreinigung

Die Aufreinigung der in Schritt B) erhaltenen Zielverbindung kann automatisch oder manuell vorgenommen werden. Zur manuellen Aufreinigung wird die Lösung so auf eine konditionierte SPE-Kartusche (SPE = Festphasenextraktion) aufgetragen (z. B. PSH+ oder SCX von Machery Nagel®, ähnliche Kartuschen sind von anderen Anbietern erhältlich), dass die Zielverbindung gebunden wird und die Nebenprodukte abgetrennt werden können. Die SPE-Kartusche wird mit sogenannten Waschlösungen gespült (H₂O, Acetonitril (ACN), verdünnte Säuren wie Essigsäure (AcOH)) und die aufgereinigte, freie Zielverbindung wird mit einem geeigneten Eluenten (z.B. 0,5 M wässrige NaOH oder 0,4 M NH₃ in H₂O/ACN) eluiert (wobei andere Eluentenlösungen möglich sind).

Dieses Eluat wird so mit einem Puffergemisch (Tris-Base, FAD-Lösung) versetzt, dass der pH-Wert der Lösung bei ca. 8-9 liegt und die Tris-Konzentration bei 50 mM, die FAD-Konzentration bei 10 µM (wobei andere Puffergemische möglich sind, FAD = Flavin-Adenin-Dinukleotid, das aber nicht zwingend notwendig ist). Diese Lösung wird langsam über eine Kartusche gegeben, welche mit 0,1-2 g immobilisierter D-α-Aminosäure-Oxidase (D-AAO) gefüllt ist.

Man erhält eine Verbindung der Formel X mit R₅ gleich Hydroxy in ca. 30 bis 50 % zerfallskorrigierter Ausbeute (10 bis 15 % nicht-zerfalls-korrigiert, bezogen auf gebildetes [¹¹C]HCN, wobei auch höhere Ausbeuten möglich sind), in > 95% radiochemischer Reinheit, und einem ee-Wert von 100 %. Ohne enzymatische Aufreiniung wird die Aminosäure mit einem ee-Wert von 70 bis 90 % erhalten.

### Beispiel 1: Synthese von L-[1-¹¹C]-Phenylalanin (Verbindung X-11)

### A) Markierung mit Kohlenstoff-11

In ein kleines Reaktorvial (V=1,3 mL) wurden der Phenylalanin-Sulfinylimin-Präkursor (*S,E*)-2-methyl-*N*-(2-phenylethylidene)propane-2-sulfinamide (Formel III-a-11, 2,1 mg) 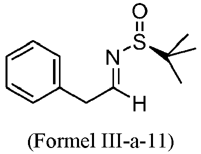 und Cäsiumfluorid (2,1 mg, 1,5 eq), gelöst in 450 µL eines Lösungsmittelgemisches, bestehend aus 1,4-Dioxan und Methanol (Mischungsverhältnis 8 : 2, bezogen auf das Volumen), vorgelegt. Das radiomarkierte, gasförmige [¹¹C]HCN wurde mit einem Trägergasstrom (Mix aus N₂, H₂ und NH₃) von ca. 65-70 mL/min von unten in dieses Reaktorvial für 8 min bei Raumtemperatur eingeleitet. Es entstand das [1-¹¹C]Pheirylalanin-Nitril als Zwischenverbindung mit Markierungsraten von 73 ± 3 % des eingesetzten [¹¹C]HCN.

### B) Hydrolyse

Als nächstes wurde konzentrierte HCl (600 µl) in dieses Reaktorvial gegeben und die Reaktionslösung für 5 min bei 150 °C erhitzt. Nach beendeter Hydrolyse wurde die Lösung in ein 10 ml-Vial gegeben, in welchem 2,5 ml einer wässrigen 1 M K₃PO₄-Lösung vorgelegt wurde, so dass der pH-Wert der Lösung auf pH = 2-2,5 eingestellt wurde.

### C) Aufreinigung

Die Lösung wurde auf eine konditionierte SPE-Kartusche aufgetragen (z.B. PSH+ von Machery Nagel), 4 ml/min. Die SPE-Kartusche wurde sukzessive mit 2 ml 3 % Essigsäure (AcOH), 2 ml Acetonitril (ACN) und 5 ml H₂O gespült und das [1-¹¹C]-Phenylalanin wurde mit 1,5 ml 0,5 M wässriger NaOH eluiert. Dieses Eluat wurde mit 500 µl eines Tris-Puffers (500 mM Tris-Base, 50 µM Flavin-Adenin-Dinukleotid, pH 7,5) versetzt und die Lösung wurde langsam über eine Kartusche gegeben, welche mit 0,5 g immobilisierter D-Alphaaminosäureoxidase (D-AAO) gefüllt war.

Es wurde L-[1-¹¹C]-Phenylalanin in ca. 30 ± 3 % zeitkorrigierter Ausbeute (bezogen auf gebildetes [¹¹C]HCN), in > 95 % radiochemischer Reinheit, und einem *ee*-Wert von 100% erhalten.

### Beispiel 2: Synthese von L-[1-¹¹C]-Tyrosin (Verbindung X-12)

### A) Markierung mit Kohlenstoff-11

In ein kleines Reaktorvial (V=1,3 mL) wurden der Tyrosin-Sulfinylimin-Präkursor (*S,E*)*-N-*(2-(4-(4-methoxybenzyloxy)phenyl)ethylidene)-2-methylpropane-2-sulfinamide (Formel III-a-12, Schutzgruppe p-Methoxybenzyl, 2,7 mg) 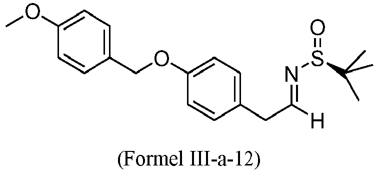 und Cäsiumfluorid (1,7 mg, 1,5 eq), gelöst in 450 µL eines Lösungsmittelgemisches, bestehend aus 1,4-Dioxan und Methanol (Mischungsverhältnis 8 : 2, bezogen auf das Volumen), vorgelegt. Das radiomarkierte, gasförmige [¹¹C]HCN wurde mit einem Trägergasstrom (Mix aus N₂, H₂ und NH₃) von ca. 65-70 mL/min von unten in dieses Reaktorvial für 8 min bei Raumtemperatur eingeleitet. Es entstand das para-methoxybenzylgeschützte [1-¹¹C]Tyrosin-Nitril als Zwischenverbindung mit Markierungsraten von 88 ± 5 % des eingesetzten [¹¹C]HCN.

### B) Hydrolyse und Abspaltung der Schutzgruppe

Das Reaktorvial wurde nun von außen erhitzt und Wasserstoffgas wurde durch die Reaktionslösung durchgeleitet, wodurch die Lösung etwas eingeengt wurde. Als nächstes wurde konzentrierte HCl (800 µl) in dieses Reaktorvial gegeben und die Reaktionslösung für 5 min bei 150 °C erhitzt. Nach beendeter Hydrolyse wurde die Lösung mit 3 ml einer wässrigen 1 M K₃PO₄-Lösung versetzt, so dass der pH-Wert der Lösung auf pH = 2-2,5 eingestellt wurde.

### C) Aufreinigung

Die Aufreinigung wurde wie in Beispiel 1 durchgeführt. Man erhielt L-[1-¹¹C]-Tyrosin in ca. 39 ± 6 % zeitkorrigierter Ausbeute (bezogen auf gebildetes [¹¹C]HCN), in > 95% radiochemischer Reinheit, und einem *ee*-Wert von 100%.

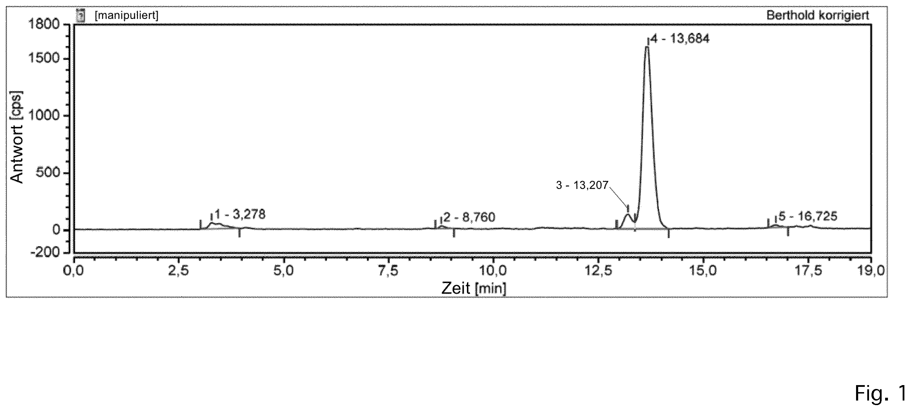

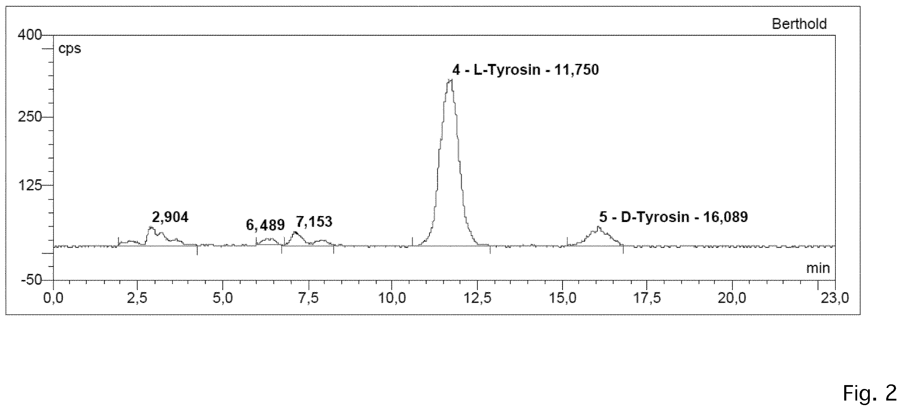

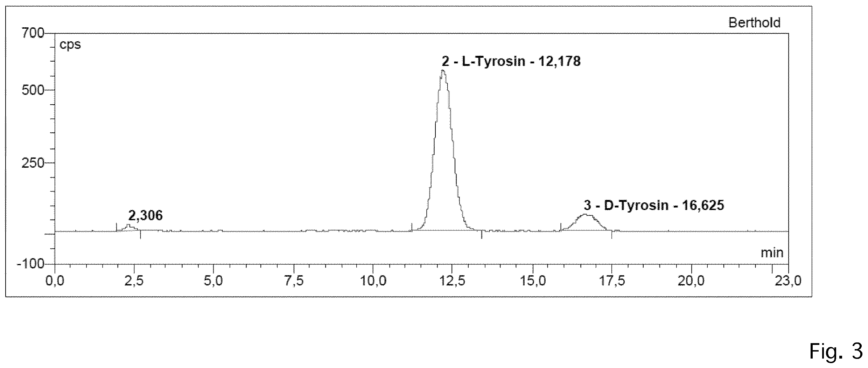

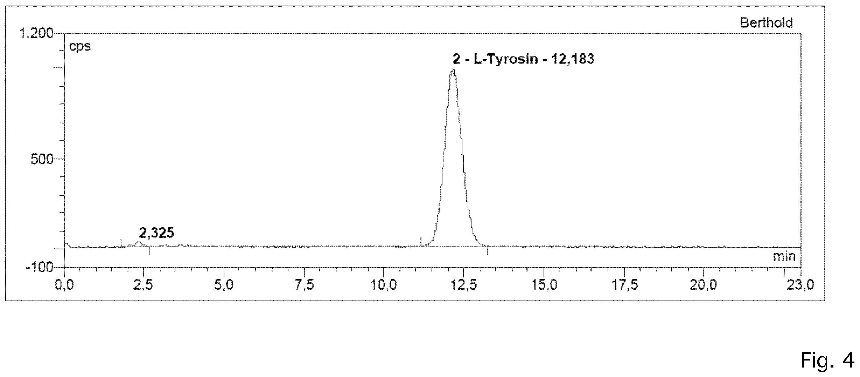

Die Figuren 1 bis 4 zeigen radiochemische HPLC-Chromatogramme, die die enantioselektive Herstellung der Zielverbindung X-12 bestätigen. Fig. 1 zeigt die Ergebnisse der Markierungsreaktion. Bei 13,207 min findet man das (S)-N-((R)-1-cyano-2-(4-(4-methoxybenzyloxy)phenyl)ethyl)-2-methylpropane-2-sulfinamid, bei 13,684 min findet man das (S)-N-((S)-1-cyano-2-(4-(4-methoxybenzyloxy)phenyl)ethyl)-2-methylpropane-2-sulfinamid. Das bestätigt, dass eines der beiden Diastereomere stark bevorzugt gebildet wird. Fig. 2 zeigt die Reaktionsprodukte nach der Hydrolyse und vor der Aufreinigung. Es ist zu erkennen, dass die Zielverbindung im hohen Überschuss zum anderen Enantiomer (D-Tyrosin) vorliegt. Fig. 3 zeigt die Ergebnisse der Aufreinigung mit einer SPE-Kartusche und Fig. 4 die Ergebnisse der enzymatischen Aufreinigung. Es ist zu erkennen, dass durch die Reinigungsschritte die Enantiomerenreinheit weiter erhöht wurde.

### Beispiel 3: Synthese von L-[1-¹¹C]-Leucin (Verbindung X-13)

### A) Markierung mit Kohlenstoff-11

In ein kleines Reaktorvial (V=1,3 mL) wurden der Leucin-Sulfinylimin-Präkursor (*S,E*)-2-methyl-*N*-(3-methylbutylidene)propane-2-sulfinamide (Formel III-a-13, 2,0 mg) 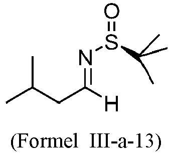 und Cäsiumfluorid (2,4 mg, 1,5 eq), gelöst in 450 µL eines Lösungsmittelgemisches bestehend aus 1,4-Dioxan und Methanol (Mischungsverhältnis 8 : 2, bezogen auf das Volumen), vorgelegt. Das radiomarkierte, gasförmige [¹¹C]HCN wurde mit einem Trägergasstrom (Mix aus N₂, H₂ und NH₃) von ca. 65-70 mL/min von unten in dieses Reaktorvial für 8 min bei Raumtemperatur eingeleitet. Es entstand das [1-¹¹C]Leucin-Nitril als Zwischenverbindung mit Markierungsraten von 70±10% des eingesetzten [¹¹C]HCNs.

### B) Hydrolyse

Die Hydrolyse wurde wie in Beispiel 1 durchgeführt, außer dass die Hydrolysetemperatur 130 °C betrug.

### C) Aufreinigung

Die Aufreinigung wurde wie in Beispiel 1 durchgeführt. Man erhielt L-[1-¹¹C]-Leucin in ca. 35 ± 5 % zeitkorrigierter Ausbeute (bezogen auf gebildetes [¹¹C]HCN), in > 95% radiochemischer Reinheit, und einem *ee*-Wert von 100 %.

### Beispiel 4: Synthese von L-[1-¹¹C]-Valin (Verbindung X-14)

### A) Markierung mit Kohlenstoff-11

In ein kleines Reaktorvial (V=1,3 mL) wurden der Valin-Sulfinylimin-Präkursor (*S*,*E*)-2-methyl-*N*-(2-methylpropylidene)propane-2-sulfinamide (Formel III-a-14 1,8 mg) 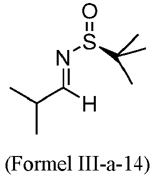 und Cäsiumfluorid (2,3 mg, 1,5 eq), gelöst in 450 µL eines Lösungsmittelgemisches, bestehend aus 1,4-Dioxan und Methanol (Mischungsverhältnis 8 : 2, bezogen auf das Volumen), vorgelegt. Das radiomarkierte, gasförmige [¹¹C]HCN wurde mit einem Trägergasstrom (Mix aus N₂, H₂ und NH₃) von ca. 65-70 mL/min von unten in dieses Reaktorvial für 8 min bei Raumtemperatur eingeleitet. Es entstand das [1-¹¹C]Valin-Nitril als Zwischenverbindung mit Markierungsraten von 70 ± 3 % des eingesetzten [¹¹C]HCN.

### B) Hydrolyse

Die Hydrolyse wurde wie in Beispiel 1 durchgeführt.

### C) Aufreinigung

Die Aufreinigung wurde wie in Beispiel 1 durchgeführt. Man erhielt L-[1-¹¹C]-Valin in ca. 18 ± 6 % zeitkorrigierter Ausbeute (bezogen auf gebildetes [¹¹C]HCN), in > 95 % radiochemischer Reinheit, und einem *ee*-Wert von 100%.

### Beispiel 5: Synthese von L-[1-¹¹C] Alanin (Verbindung X-15)

In ein kleines Reaktorvial (V=1,3 mL) wurden der Alanin-Sulfinylimin-Präkursor (*S*,*E*)-*N*-ethylidene-2-methylpropane-2-sulfinamide (Formel III-a-15 1,8 mg) 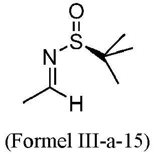 und Cäsiumfluorid (2,8 mg, 1,5 eq), gelöst in 450 µL eines Lösungsmittelgemisches, bestehend aus 1,4-Dioxan und Methanol (Mischungsverhältnis 8 : 2, bezogen auf das Volumen), vorgelegt. Das radiomarkierte, gasförmige [¹¹C]HCN wurde mit einem Trägergasstrom (Mix aus N₂, H₂ und NH₃) von ca. 65-70 mL/min von unten in dieses Reaktorvial für 8 min bei Raumtemperatur eingeleitet. Es entstand das [1-¹¹C]Alanin-Nitril als Zwischenverbindung mit Markierungsraten von 76 ± 5 % des eingesetzten [¹¹C]HCN.

### Beispiel 6: Synthese von L-[1-¹¹C]-Serin (Verbindung X-16)

In ein kleines Reaktorvial (V=1,3 mL) wurden der Serin-Sulfinylimin-Präkursor [(S,E)-N-(2-(4-methoxybenzyloxy)ethylidene)-2-methylpropane-2-sulfinamide (Verbindung III-a-16, Schutzgruppe p-Methoxybenzyl, 2,4 mg) 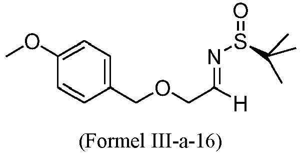 und Cäsiumfluorid (1,9 mg, 1,5 eq), gelöst in 450 µL eines Lösungsmittelgemisches, bestehend aus 1,4-Dioxan und Methanol (Mischungsverhältnis 8 : 2, bezogen auf das Volumen), vorgelegt. Das radiomarkierte, gasförmige [¹¹C]HCN wurde mit einem Trägergasstrom (Mix aus N₂, H₂ und NH₃) von ca. 65-70 mL/min von unten in dieses Reaktorvial für 8 min bei Raumtemperatur eingeleitet. Es entstand das para-methoxybenzylgeschützte [1-¹¹C]Serin-Nitril als Zwischenverbindung mit Markierungsraten von 78±5% des eingesetzten [¹¹C]HCN.

## Patentansprüche

1. Verwendung eines Präkursors für die Herstellung von Kohlenstoff-11 markierten Aminosäuren oder Derivaten davon, wobei der Präkursor eine Verbindung der Formel I ist: 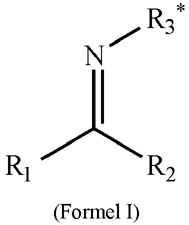 wobei
R₁ und R₂ unabhängig voneinander aus der Gruppe ausgewählt sind, die Wasserstoff, unsubstituiertes oder substituiertes C₁-C₆-Alkyl, das gegebenenfalls durch Einlagerung zumindest einer Gruppe X in die Kohlenstoffkette modifiziert sein kann, unsubstituiertes oder substituiertes C₂-C₆-Alkenyl, das gegebenenfalls durch Einlagerung zumindest einer Gruppe X in die Kohlenstoffkette modifiziert sein kann, substituiertes oder unsubstituiertes Alkylaryl, substituiertes oder unsubstituiertes Arylalkyl, substituiertes oder unsubstituiertes Aryl und substituiertes oder unsubstituiertes Heteroaryl umfasst,
R₃ ein chirales Auxiliar ist, das aus der Gruppe ausgewählt ist, die substituiertes oder unsubstituiertes C₁-C₆-Alkylsulfinyl, substituiertes oder unsubstituiertes Arylsulfinyl, substituiertes oder unsubstituiertes Arylalkyl und substituiertes oder unsubstituiertes Arylglycinol umfasst,
X aus der Gruppe ausgewählt ist, die Sauerstoff, Schwefel, -SO-, -SO₂- und -N(R₁₀)- umfasst,
R₁₀ Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst und
die Reste gegebenenfalls ungeschützt oder geschützt sind.

2. Verwendung nach Anspruch 1, **dadurch gekennzeichnet, dass** der Präkursor eine Verbindung der Formel II-a oder II-b ist: 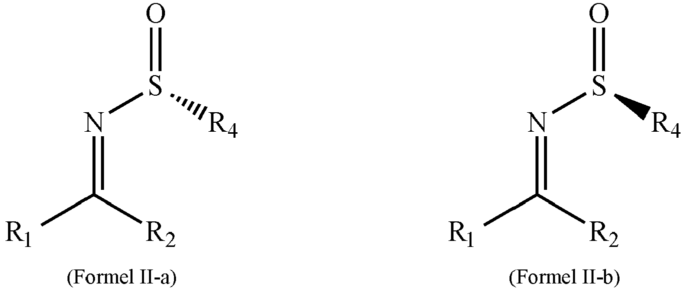 wobei R₁ und R₂ wie in Anspruch 1 definiert sind und R₄ aus der Gruppe ausgewählt ist, die unsubstituiertes oder substituiertes C₁-C₆-Alkyl und substituiertes oder unsubstituiertes Aryl umfasst.

3. Verwendung nach Anspruch 2, **dadurch gekennzeichnet, dass** R₁ aus der Gruppe ausgewählt ist, die unsubstituiertes oder substituiertes C₁-C₆-Alkyl, substituiertes oder unsubstituiertes Aryl und substituiertes oder unsubstituiertes Heteroaryl umfasst; R₂ Wasserstoff ist; und R₄ wie in Anspruch 2 definiert ist.

4. Verwendung nach einem der vorstehenden Ansprüche, **dadurch gekennzeichnet, dass** R₁ aus der Gruppe ausgewählt ist, die Methyl, geschütztes oder ungeschütztes Hydroxymethyl, Ethyl, n-Propyl, iso-Propyl, n-Butyl, iso-Butanyl, sec-Butyl, tert-Butyl, Phenyl, geschütztes oder ungeschütztes Hydroxyphenyl, Benzyl und geschütztes oder ungeschütztes Hydroxybenzyl umfasst, und R₂ Wasserstoff ist.

5. Verfahren zur diastereoselektiven Markierung eines Präkursors der Formel I: 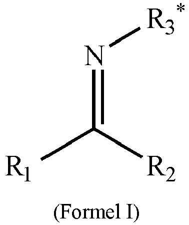 wobei
R₁ und R₂ unabhängig voneinander aus der Gruppe ausgewählt sind, die Wasserstoff, unsubstituiertes oder substituiertes C₁-C₆-Alkyl, das gegebenenfalls durch Einlagerung zumindest einer Gruppe X in die Kohlenstoffkette modifiziert sein kann, unsubstituiertes oder substituiertes C₂-C₆-Alkenyl, das gegebenenfalls durch Einlagerung zumindest einer Gruppe X in die Kohlenstoffkette modifiziert sein kann, substituiertes oder unsubstituiertes Alkylaryl, substituiertes oder unsubstituiertes Arylalkyl, substituiertes oder unsubstituiertes Aryl und substituiertes oder unsubstituiertes Heteroaryl umfasst,
R₃ ein chirales Auxiliar ist, das aus der Gruppe ausgewählt ist, die substituiertes oder unsubstituiertes C₁-C₆-Alkylsulfinyl, substituiertes oder unsubstituiertes Arylsulfinyl, substituiertes oder unsubstituiertes Arylalkyl und substituiertes oder unsubstituiertes Arylglycinol umfasst,
X aus der Gruppe ausgewählt ist, die Sauerstoff, Schwefel, -SO-, -SO₂- und -N(R₁₀)- umfasst,
R₁₀ Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst und
die Reste gegebenenfalls ungeschützt oder geschützt sind,
**dadurch gekennzeichnet, dass** der Präkursor mit einem Kohlenstoff-11-markierten Synthon zu einer Kohlenstoff-11-markierten Verbindung, die eine Kohlenstoff-11-markierte Carbonylgruppe aufweist, umgesetzt wird.

6. Verfahren nach Anspruch 5, **dadurch gekennzeichnet, dass** die Kohlenstoff-11 markierte Verbindung eine Verbindung der Formel X ist: 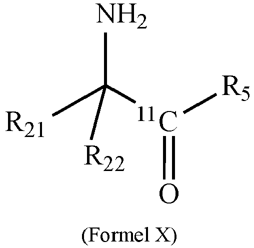 wobei
R₂₁ und R₂₂ die in den Ansprüchen 1 bis 4 für R₁ und R₂ angegebenen Bedeutungen haben,
R₅ aus der Gruppe ausgewählt ist, die OR₆ und NR₇R₈ umfasst,
R₆, R₇ und R₈ unabhängig voneinander aus der Gruppe ausgewählt sind, die Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst,
wobei ein Präkursor gemaß einem der Ansprüche 1 bis 4 mit einem Kohlenstoff-11-markierten Synthon zu der Verbindung der Formel X umgesetzt wird.

7. Verfahren nach Anspruch 5 oder Anspruch 6, **dadurch gekennzeichnet, dass** das Kohlenstoff-11-markierte Synthon [¹¹C]R₃₁CN ist, wobei R₃₁ aus der Gruppe ausgewählt ist, die Wasserstoff, ein Alkalimetall wie Lithium, Natrium oder Kalium, Acetyl und Alkylsilyl umfasst.

8. Verfahren nach einem der Ansprüche 5 bis 7, **dadurch gekennzeichnet, dass** der Präkursor mit dem Kohlenstoff-11-markierten Synthon zu einer Verbindung der Formel V umgesetzt wird: 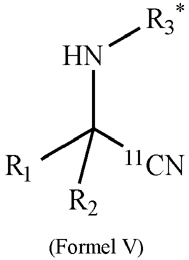 wobei R₁, R₂ und R₃ die in Anspruch 5 angegebenen Bedeutungen aufweisen.

9. Verfahren nach Anspruch 8, **dadurch gekennzeichnet, dass** die Verbindung der Formel V mittels Alkoholyse oder Hydrolyse in eine Verbindung der Formel XI überführt wird, 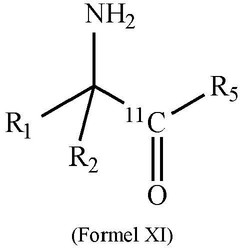 wobei R₁ und R₂ die in den Ansprüchen 1 bis 4 angegebenen Bedeutungen haben und R₅ wie in Anspruch 6 definiert ist, mit der Maßgabe, dass R₅ nicht OH ist.

10. Verfahren nach Anspruch 9, **dadurch gekennzeichnet, dass** die Verbindung der Formel XI mittels Hydrolyse in eine Verbindung der Formel XII überführt wird 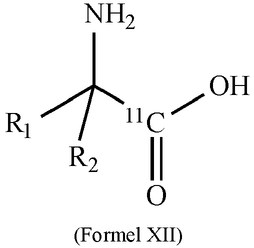 wobei R₁ und R₂ die in den Ansprüchen 1 bis 4 angegebenen Bedeutungen haben.

11. Verfahren nach einem der Ansprüche 6 bis 8, **dadurch gekennzeichnet, dass** zur Herstellung einer Verbindung der Formel XIII: 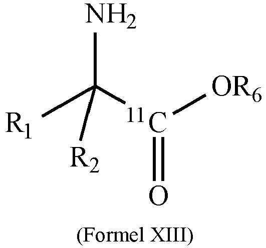 ein Präkursor der Formel II 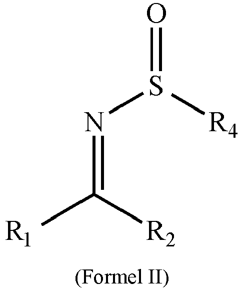 verwendet wird, wobei R₁ und R₂ die in den Ansprüchen 1 bis 4 angegebenen Bedeutungen haben, R₆ die in Anspruch 6 angegebenen Bedeutungen hat und R₄ die in Anspruch 2 angegebene Bedeutung hat.

12. Verfahren nach Anspruch 11, **dadurch gekennzeichnet, dass** eine Verbindung der Formel II mit dem Kohlenstoff-11-markierten Synthon zu einer Verbindung der Formel XIV umgesetzt wird: 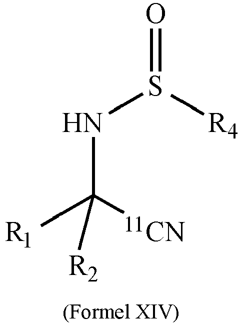 wobei R₁ und R₂ die in Anspruch 6 angegebenen Bedeutungen haben und R₄ die in Anspruch 2 angegebene Bedeutung hat.

13. Verfahren nach Anspruch 12, **dadurch gekennzeichnet, dass** die Verbindung der Formel XIV mittels Alkoholyse in eine Verbindung der Formel XV überführt wird: 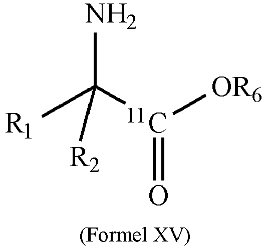 wobei R₁ und R₂ die in den Ansprüchen 1 bis 4 angegebenen Bedeutungen haben und R₆ wie in Anspruch 6 definiert ist, mit der Maßgabe, dass R₆ nicht Wasserstoff ist.

14. Verfahren nach Anspruch 12, **dadurch gekennzeichnet, dass** die Verbindung der Formel XIV mittels Hydrolyse in eine Verbindung der Formel XVI überführt wird: 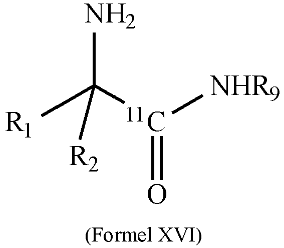 wobei R₁ und R₂ die in den Ansprüchen 1 bis 4 angegebenen Bedeutungen haben und R₉ aus der Gruppe ausgewählt ist, die Wasserstoff, unsubstituiertes oder substituiertes C₁-C₆-Alkyl, unsubstituiertes oder substituiertes C₂-C₆-Alkenyl, substituiertes oder unsubstituiertes Aryl und substituiertes oder unsubstituiertes Heteroaryl umfasst.

15. Verfahren nach Anspruch 13 oder Anspruch 14, **dadurch gekennzeichnet, dass** die Verbindung der Formel XV oder XVI mittels Hydrolyse in eine Verbindung der Formel XII überführt wird: 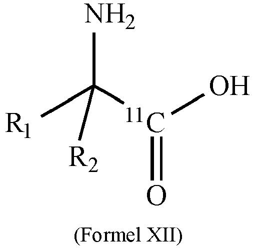 wobei R₁ und R₂ die in den Ansprüchen 1 bis 4 angegebenen Bedeutungen haben.

16. Verfahren nach einem der vorstehenden Ansprüche, **dadurch gekennzeichnet, dass** die Reste R₁ und R₂ einer Verbindung der Formeln XI, XII, XIII, XV oder XVI den Resten R₂₁ und/oder R₂₂ der Verbindung X entsprechen, sofern die Reste R₁ und R₂ ungeschützt sind, oder dass der Rest R₁ einer Verbindung der Formeln XI, XII, XIII, XV oder XVI in den Rest R₂₁ der Verbindung X durch Abspalten einer oder mehrerer Schutzgruppen überführt wird und/oder dass der Rest R₂ einer Verbindung der Formeln XI, XII, XIII, XV oder XVI in den Rest R₂₂ der Verbindung X durch Abspalten einer oder mehrerer Schutzgruppen überführt wird.

17. Kit zur enantioselektiven Herstellung einer Kohlenstoff-11-markierten Aminosäure oder eines Derivates davon, aufweisend einen Präkursor nach einem der Ansprüche 1 bis 4, ein oder mehrere Lösungsmittel, einen oder mehrere Hilfsstoffe und eine oder mehrere Reinigungskartuschen.

## Claims

1. Use of a precursor for the preparation of carbon-11 labelled amino acids or derivatives thereof, wherein the precursor is a compound of formula I: 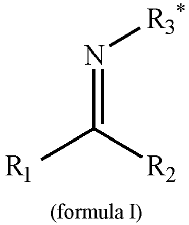 wherein
R₁ and R₂ are independently selected from the group comprising hydrogen, unsubstituted or substituted C₁-C₆alkyl that can optionally be modified by inserting at least one group X into the carbon chain, unsubstituted or substituted C₂-C₆alkenyl that can optionally be modified by inserting at least one group X into the carbon chain, substituted or unsubstituted alkyl aryl, substituted or unsubstituted aryl alkyl, substituted or unsubstituted aryl, and substituted or unsubstituted heteroaryl,
R₃ is a chiral auxiliary selected from the group comprising substituted or unsubstituted C₁-C₆alkyl sulphinyl, substituted or unsubstituted aryl sulphinyl, substituted or unsubstituted aryl alkyl, and substituted or unsubstituted aryl glycinol,
X is selected from the group comprising oxygen, sulphur, -SO-, -SO₂-, and -N(R₁₀)-,
R₁₀ comprises hydrogen, unsubstituted or substituted C₁-C₆alkyl, unsubstituted or substituted C₂-C₆alkenyl, substituted or unsubstituted aryl, and substituted or unsubstituted heteroaryl; and
the residues are optionally unprotected or protected.

2. Use according to claim 1 **characterized in that** the precursor is a compound of formula II-a or II-b: 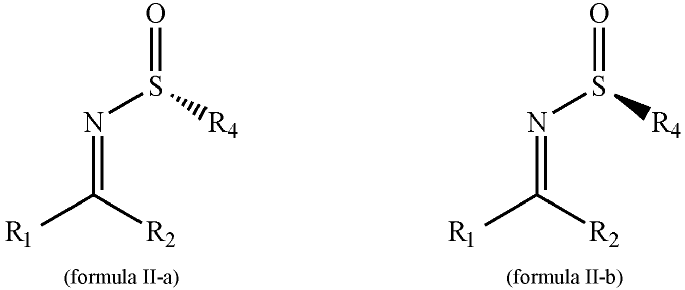 wherein R₁ and R₂ are as defined in claim 1, and R₄ is selected from the group comprising unsubstituted or substituted C₁-C₆alkyl, and substituted or unsubstituted aryl.

3. Use according to claim 2 **characterized in that** R₁ is selected from the group comprising unsubstituted or substituted C₁-C₆alkyl, substituted or unsubstituted aryl, and substituted or unsubstituted heteroaryl; R₂ is hydrogen; and R₄ is as defined in claim 2.

4. Use according to any one of the preceding claims **characterized in that** R₁ is selected from the group comprising methyl, protected or unprotected hydroxymethyl, ethyl, n-propyl, iso-propyl, n-butyl, iso-butanyl, sec-butyl, tert-butyl, phenyl, protected or unprotected hydroxyphenyl, benzyl, and protected or unprotected hydroxybenzyl, and R₂ is hydrogen.

5. A method for diastereo-selective labelling a precursor of formula I: 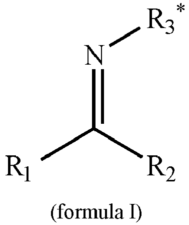 wherein
R₁ and R₂ are independently selected from the group comprising hydrogen, unsubstituted or substituted C₁-C₆alkyl that can optionally be modified by inserting at least one group X into the carbon chain, unsubstituted or substituted C₂-C₆alkenyl that can optionally be modified by inserting at least one group X into the carbon chain, substituted or unsubstituted alkyl aryl, substituted or unsubstituted aryl alkyl, substituted or unsubstituted aryl, and substituted or unsubstituted heteroaryl,
R₃ is a chiral auxiliary selected from the group comprising substituted or unsubstituted C₁-C₆alkyl sulphinyl, substituted or unsubstituted aryl sulphinyl, substituted or unsubstituted aryl alkyl and substituted or unsubstituted aryl glycinol,
X is selected from the group comprising oxygen, sulphur, -SO-, -SO₂-, and -N(R₁₀)-,
R₁₀ comprises hydrogen, unsubstituted or substituted C₁-C₆alkyl, unsubstituted or substituted C₂-C₆alkenyl, substituted or unsubstituted aryl, and substituted or unsubstituted heteroaryl; and
the residues are optionally unprotected or protected,
**characterized in that** the precursor is reacted with a carbon-11 labelled synthon to a carbon-11 labelled compound having a carbon-11 labelled carbonyl group.

6. The method according to claim 5 **characterized in that** the carbon-11 labelled compound is a compound of formula X: 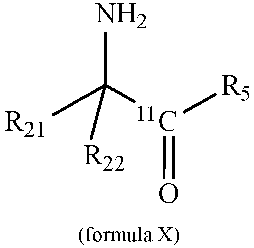 wherein
R₂₁ and R₂₂ have the meanings given in claims 1 to 4 for R₁ and R₂,
R₅ is selected from the group comprising OR₆, and NR₇R₈,
R₆, R₇ and R₈ are independently selected from the group comprising hydrogen, unsubstituted or substituted C₁-C₆alkyl, unsubstituted or substituted C₂-C₆alkenyl, substituted or unsubstituted aryl, and substituted or unsubstituted heteroaryl,
wherein a precursor according to any one of claims 1 to 4 is reacted with a carbon-11 labelled synthon to the compound of formula X.

7. The method according to claim 5 or claim 6 **characterized in that** the carbon-11 labelled synthon is [¹¹C]R₃₁CN wherein R₃₁ is selected from the group comprising hydrogen, an alkaline metal such as lithium, sodium, or potassium, acetyl, and alkyl silyl.

8. The method according to any one of claims 5 to 7 **characterized in that** the precursor is reacted with the carbon-11 labelled synthon to a compound of formula V: 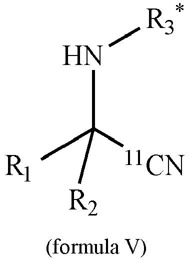 wherein R₁, R₂ and R₃ have the meanings given in claim 5.

9. The method according to claim 8 **characterized in that** the compound of formula V is converted to a compound of formula XI by means of alcoholysis, or hydrolysis, 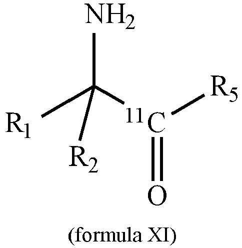 wherein R₁ and R₂ have the meanings given in claims 1 to 4, and R₅ is as defined in claim 6, with the proviso that R₅ is not OH.

10. The method according to claim 9 **characterized in that** the compound of formula XI is converted to a compound of formula XII by means of hydrolysis 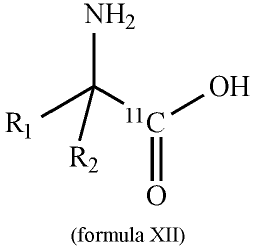 wherein R₁ and R₂ have the meanings given in claims 1 to 4.

11. The method according to any one of claims 6 to 8 **characterized in that** for the preparation of a compound of formula XIII: 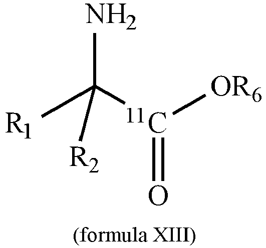 a precursor of formula II 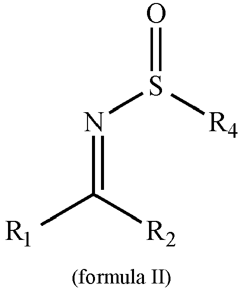 is used, wherein R₁ and R₂ have the meanings given in claims 1 to 4, R₆ has the meanings given in claim 6, and R₄ has the meaning given in claim 2.

12. The method according to claim 11 **characterized in that** a compound of formula II is reacted with the carbon-11 labelled synthon to a compound of formula XIV: 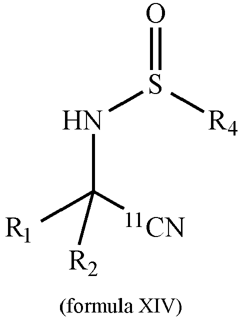 wherein R₁ and R₂ have the meanings given in claim 6, and R₄ has the meaning given in claim 2.

13. The method according to claim 12 **characterized in that** the compound of formula XIV is converted to a compound of formula XV by means of alcoholysis: 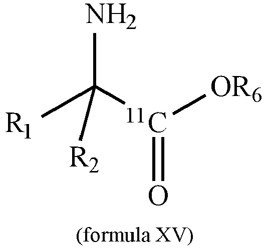 wherein R₁ and R₂ have the meanings given in claims 1 to 4, and R₆ is as defined in claim 6, with the proviso that R₆ is not hydrogen.

14. The method according to claim 12 **characterized in that** the compound of formula XIV is converted into a compound of formula XVI by means of hydrolysis: 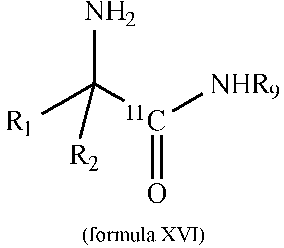 wherein R₁ and R₂ have the meanings given in claims 1 to 4, and R₉ is selected from the group comprising hydrogen, unsubstituted or substituted C₁-C₆alkyl, unsubstituted or substituted C₂-C₆alkenyl, substituted or unsubstituted aryl, and substituted or unsubstituted heteroaryl.

15. The method according to claim 13 or claim 14 **characterized in that** the compound of formula XV or XVI is converted into a compound of formula XII by means of hydrolysis: 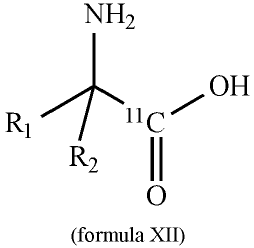 wherein R₁ and R₂ have the meanings given in claims 1 to 4.

16. The method according to any one of the preceding claims **characterized in that** residues R₁ and R₂ of a compound of formulae XI, XII, XIII, XV, or XVI correspond to residues R₂₁ and/or R₂₂ of compound X, provided that residues R₁ and R₂ are unprotected, or that residue R₁ of a compound of formulae XI, XII, XIII, XV, or XVI is converted into the residue R₂₁ of compound X by cleaving one or more protecting groups, and/or that residue R₂ of a compound of formulae XI, XII, XIII, XV, or XVI is converted into residue R₂₂ of compound X by cleaving one or more protecting groups.

17. A kit for enantio-selective preparation of a carbon-11 labelled amino acid or a derivative thereof having a precursor according to any one of claims 1 to 4, one or more solvents, one or more excipients, and one or more cleaning cartridges.

## Revendications

1. Utilisation d'un précurseur pour la fabrication d'acides aminés marqués au carbone 11 ou de leurs dérivés, ledit précurseur étant une liaison de la formule I : 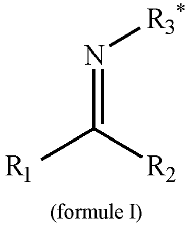
R₁ et R₂ étant choisis indépendamment l'un de l'autre dans le groupe qui comprend hydrogène, alkyle C₁-C₆ non-substitué ou substitué pouvant être modifié le cas échéant par intercalation d'au moins un groupe X dans la chaîne carbonée, alkényle C₂-C₆ non-substitué ou substitué pouvant être modifié le cas échéant par intercalation d'au moins un groupe X dans la chaîne carbonée, alkylaryle substitué ou non-substitué, arylalkyle substitué ou non-substitué, aryle substitué ou non-substitué et hétéroaryle substitué ou non-substitué,
R₃ étant un auxiliaire chiral choisi dans le groupe qui comprend alkylsulfinyle C₁-C₆ substitué ou non-substitué, arylsulfinyle substitué ou non-substitué, arylalkyle substitué ou non-substitué et arylglycinol substitué ou non-substitué,
X étant choisi dans le groupe qui comprend oxygène, soufre, -SO-, -SO₂- et -N(R₁₀)-,
R₁₀ comprenant hydrogène, alkyle C₁-C₆ non-substitué ou substitué, alkényle C₂-C₆ non-substitué ou substitué, aryle substitué ou non-substitué et hétéroaryle substitué ou non-substitué,
les restes étant, le cas échéant, non-protégés ou protégés.

2. Utilisation selon la revendication 1 **caractérisée en ce que** ledit précurseur est une liaison de la formule II-a ou II-b : 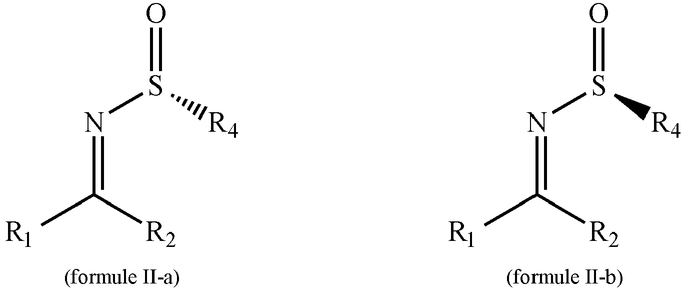 R₁ et R₂ étant définis selon la revendication 1, et R₄ étant choisi dans le groupe qui comprend alkyle C₁-C₆ non-substitué ou substitué et aryle substitué ou non-substitué.

3. Utilisation selon la revendication 2 **caractérisée en ce que** R₁ est choisi dans le groupe qui comprend alkyle C₁-C₆ non-substitué ou substitué, aryle substitué ou non-substitué et hétéroaryle substitué ou non-substitué ; R₂ étant l'hydrogène ; et R₄ étant défini selon la revendication 2.

4. Utilisation selon l'une des revendications précédentes **caractérisée en ce que** R₁ est choisi dans le groupe qui comprend méthyle, hydroxyméthyle protégé ou non, éthyle, n-propyle, iso-propyle, n-butyle, iso-butanyle, sec-butyle, tert-butyle, phényle, hydroxyphényle protégé ou non, benzyle et hydroxybenzyle protégé ou non, et que R₂ est l'hydrogène.

5. Procédé pour le marquage diastéréosélectif d'un précurseur de la formule I : 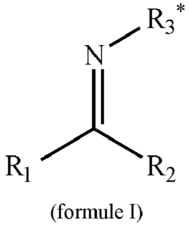
R₁ et R₂ étant choisis indépendamment l'un de l'autre dans le groupe qui comprend hydrogène, alkyle C₁-C₆ non-substitué ou substitué pouvant être modifié le cas échéant par intercalation d'au moins un groupe X dans la chaîne carbonée, alkényle C₂-C₆ non-substitué ou substitué pouvant être modifié le cas échéant par intercalation d'au moins un groupe X dans la chaîne carbonée, alkylaryle substitué ou non-substitué, arylalkyle substitué ou non-substitué, aryle substitué ou non-substitué et hétéroaryle substitué ou non-substitué,
R₃ étant un auxiliaire chiral choisi dans le groupe qui comprend alkylsulfinyle C₁-C₆ substitué ou non-substitué, arylsulfinyle substitué ou non-substitué, arylalkyle substitué ou non-substitué et arylglycinol substitué ou non-substitué,
X étant choisi dans le groupe qui comprend oxygène, soufre, -SO-, -SO₂- et -N(R₁₀)-,
R₁₀ comprenant hydrogène, alkyle C₁-C₆ non-substitué ou substitué, alkényle C₂-C₆ non-substitué ou substitué, aryle substitué ou non-substitué et hétéroaryl substitué ou non-substitué et
les restes étant, le cas échéant, non-protégés ou protégés,
**caractérisé en ce que** ledit précurseur est converti avec un synthon marqué au carbone 11 en une liaison marquée au carbone 11, laquelle présente un groupe carbonyle marqué au carbone 11.

6. Procédé selon la revendication 5 **caractérisé en ce que** ladite liaison marquée au carbone 11est une liaison de la formule X : 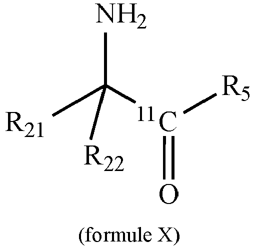
R₂₁ et R₂₂, ayant les significations données pour R₁ et R₂ dans les revendications 1 à 4,
R₅ étant choisi dans le groupe qui comprend OR₆ et NR₇R₈,
R₆, R₇ et R₈ étant choisis indépendamment dans le groupe qui comprend hydrogène, alkyle C₁-C₆ non-substitué ou substitué, alkényle C₂-C₆ non-substitué ou substitué, aryle substitué ou non-substitué et hétéroaryle substitué ou non-substitué,
un précurseur selon l'une des revendications 1 à 4 étant converti avec un synthon marqué au carbone 11 en la liaison de la formule X.

7. Procédé selon la revendication 5 ou la revendication 6 **caractérisé en ce que** ledit synthon marqué au carbone 11 est [¹¹C]R₃₁CN, R₃₁ étant choisi dans le groupe qui comprend hydrogène, un métal alcalin tel que lithium, sodium ou potassium, acétyle et alkylsilyle.

8. Procédé selon l'une des revendications 5 à 7 **caractérisé en ce que** ledit précurseur est converti avec ledit synthon marqué au carbone 11 en une liaison de la formule V : 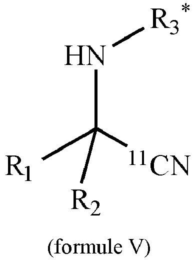 R₁, R₂ et R₃ présentant les significations indiquées à la revendication 5.

9. Procédé selon la revendication 8 **caractérisé en ce que** la liaison de ladite formule V est converti par alcoolyse ou hydrolyse en une liaison de la formule XI : 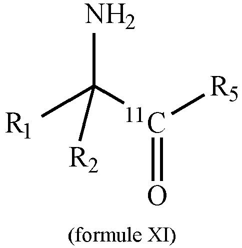 R₁ et R₂ ayant les significations indiquées aux revendications 1 à 4, et R₅ étant défini comme dans la revendication 6, à condition que R₅ ne soit pas OH.

10. Procédé selon la revendication 9 **caractérisé en ce que** la liaison de ladite formule XI est convertie par hydrolyse en une liaison de la formule XII : 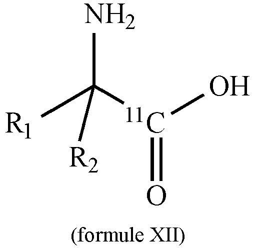 R₁ et R₂ ayant les significations indiquées aux revendications 1 à 4.

11. Procédé selon l'une des revendications 6 à 8 **caractérisé en ce que** pour l'obtention d'une liaison de la formule XIII : 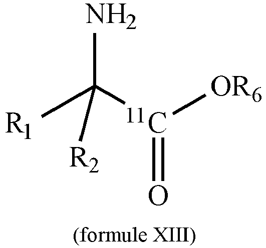 on utilise un précurseur de la formule II, 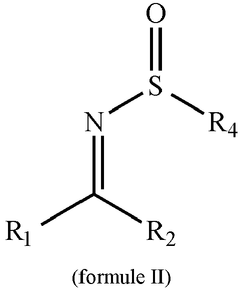 R₁ et R₂ ayant les significations indiquées aux revendications 1 à 4, R₆ ayant les significations indiquées à la revendication 6 et R₄ ayant la signification indiquée à la revendication 2.

12. Procédé selon la revendication 11 **caractérisé en ce que** qu'une liaison de ladite formule II est convertie avec ledit synthon marqué au carbone 11 en une liaison de la formule XIV : 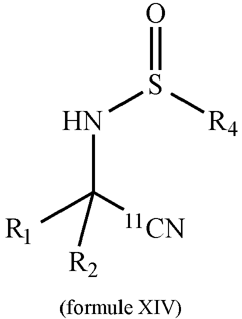 R₁ et R₂ ayant les significations indiquées à la revendication 6, et R₄ ayant la signification indiquée à la revendication 2.

13. Procédé selon la revendication 12 **caractérisé en ce que** la liaison de ladite formule XIV est convertie par alcoolyse en une liaison de la formule XV : 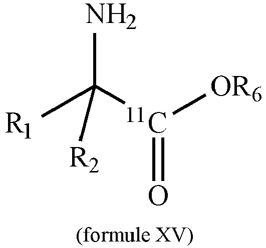 R₁ et R₂ ayant les significations indiquées aux revendications 1 à 4, et R₆ étant défini comme à la revendication 6, à condition que R₆ ne soit pas de l'hydrogène.

14. Procédé selon la revendication 12 **caractérisé en ce que** la liaison de ladite formule XIV est convertie par hydrolyse en une liaison de la formule XVI : 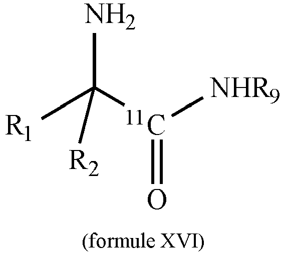 R₁ et R₂ ayant les significations indiquées aux revendications 1 à 4, et R₉ étant choisi dans le groupe qui comprend hydrogène, alkyle C₁-C₆ non-substitué ou substitué, alkényle C₂-C₆ non-substitué ou substitué, aryle substitué ou non-substitué et hétéroaryle substitué ou non-substitué.

15. Procédé selon la revendication 13 ou la revendication 14 **caractérisé en ce que** la liaison desdites formules XV et XVI est convertie par hydrolyse en une liaison de la formule XII : 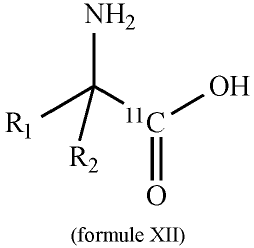 R₁ et R₂ ayant les significations indiquées aux revendications 1 à 4.

16. Procédé selon l'une des revendications précédentes **caractérisé en ce que** les restes R₁ et R₂ d'une liaison desdites formules XI, XII, XIII, XV ou XVI correspondent au restes R₂₁ et/ou R₂₂ de la liaison X, lorsque lesdits restes R₁ et R₂ sont non-protégés, ou **en ce que** ledit reste R₁ d'une liaison desdites formules XI, XII, XIII, XV ou XVI est converti en reste R₂₁ de ladite liaison X par séparation d'un ou plusieurs groupes de protection, et/ou que ledit reste R₂ d'une liaison desdites formules XI, XII, XIII, XV ou XVI est converti en reste R₂₂ de ladite liaison X par séparation d'un ou plusieurs groupes de protection.

17. Kit pour l'obtention énantiosélective d'un acide aminé marqué au carbone 11, ou d'un de ses dérivés, présentant un précurseur selon l'une des revendications 1 à 4, un ou plusieurs solvants, un ou plusieurs agents accessoires et une ou plusieurs cartouches de nettoyage.
